# EUROPEAN PATENT APPLICATION

(11) **EP 2 497 825 A1**
(43) Date of publication of application: **12.09.2012**
(21) Application number: 11001870.2
(22) Date of filing: 07.03.2011
(51) Int. Cl.: C12N 5/0735

(54) **Novel method for rapid and efficient generation of neuroectoderm cells and peripheral neurons from pluripotent stem cells**

(71) Applicant: Max-Planck-Gesellschaft zur Förderung der Wissenschaften e.V., 80539 München (DE)
(72) Inventor: Greber, Boris, 48161 Münster (DE); Schöler, Hans, 48151 Münster (DE)
(74) Representative: Vossius & Partner

(57) **Abstract**

The present invention relates to a method for generating neuroectoderm cells, the method comprising: culturing pluripotent cells for about 2 to about 6 days, wherein the expression and/or activity of one or more proteins selected from the group consisting of fibroblast growth factor receptor (FGFR), son of sevenless (SOS), growth factor receptor-bound protein 2 (GRB2), rat sarcoma protein (RAS), rat fibrosarcoma (RAF), mitogen-activated protein kinase kinase (MEK) and mitogen-activated protein kinase (MAPK) is inhibited. The present invention further relate to a method for generating neurons, the method comprising the steps for generating neuroectoderm cells in accordance with the invention and culturing the neuroectoderm cells for at least about 2 further days. Furthermore, the present invention also relates to a kit comprising means for inhibiting the expression and/or activity of one or more of the proteins selected from the group consisting of FGFR, SOS, GRB2, RAS, RAF, MEK and MAPK, and/or means inhibiting the nodal/activin/TGFbeta/SMAD2/3 signalling pathway, and/or the BMP/GDF/SMAD1/5/8 signalling pathway and, optionally, pluripotent cells.

## Description

The present invention relates to a method for generating neuroectoderm cells, the method comprising: culturing pluripotent cells for about 2 to about 6 days, wherein the expression and/or activity of one or more proteins selected from the group consisting of fibroblast growth factor receptor (FGFR), son of sevenless (SOS), growth factor receptor-bound protein 2 (GRB2), rat sarcoma protein (RAS), rat fibrosarcoma (RAF), mitogen-activated protein kinase kinase (MEK) and mitogen-activated protein kinase (MAPK) is inhibited. The present invention further relates to a method for generating neurons, the method comprising the steps for generating neuroectoderm cells in accordance with the invention and culturing the neuroectoderm cells for at least about 2 further days. Furthermore, the present invention also relates to a kit comprising means for inhibiting the expression and/or activity of one or more of the proteins selected from the group consisting of FGFR, SOS, GRB2, RAS, RAF, MEK and MAPK, and/or means inhibiting the nodal/activin/TGFbeta/SMAD2/3 signalling pathway, and/or the BMP/GDF/ SMAD1/5/8 signalling pathway and, optionally, pluripotent cells.

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Human embryonic stem cells (hESCs or hES cells) can exit the self-renewal programme at any given time and differentiate along the three germ layer lineages, i.e. mesoderm, endoderm and ectoderm. This capacity for differentiation is mediated through the action of various signaling molecules and provides numerous possibilities for regenerative medicine and for studying mechanisms of early cell fate regulation.

The TGFβ and BMP pathways, two evolutionary conserved signaling cascades, activate a set of transcription factors - SMAD 2/3 and SMAD 1/5/8, respectively, which in turn activate or repress the expression of target genes (Schmierer and Hill, 2007). The balance between these pathways has a major impact on cell fate specification in hESCs (Vallier *et al.* 2009; Greber, Lehrach and Adjaye 2008; Yao *et al.* 2006). hESCs resemble epiblast stem cells derived from pre-gastrulation mouse embryos regarding their responsiveness to extracellular signals (Tesar *et al.* 2007). Consistent with the absence of inductive signals in the anterior mouse epiblast (Murry and Keller 2008), neuroectoderm formation in hESCs is facilitated by the inhibition of TGFβ signaling (Smith *et al.* 2008), the inhibition of BMP signaling (Pera *et al.* 2004), or both (Chambers *et al.* 2009). These findings have been further discussed for example in WO 2010/096496, which describes a method for differentiating human stem cells into non-default differentiated cells, such as e.g. neural progenitor cells, comprising the inhibition of SMAD protein signalling. Also Greber *et al.* 2008³ describes the role of SMAD signalling in controlling self-renewal of human embryonic stem cells versus lineage commitment. Chambers *et al.* 2009 describe the differentiation of hESCs into neuroectoderm by the combined inhibition of TGFβ and BMP signaling, which avoids the use of embryoid bodies or stromal feeder cells.

FGF2 is a major factor involved in the self-renewal of hESCs and has been shown to activate the ERK cascade (Kang *et al.* 2005). The effects of FGF2 signaling in hESCs appear to be complex and relatively subtle. In addition to positive effects on cell survival and/or proliferation, FGF2, on the one hand, enhances the expression of the pluripotency-controlling gene NANOG (Greber *et a*/*.* 2010), in collaboration with TGFβ/SMAD2 signaling (Greber, Lehrach and Adjaye 2008; Xu *et al.* 2008; Vallier *et al*. 2009a). On the other hand, FGF2 signaling antagonises BMP signaling, a major (non-neural) differentiation-inducing pathway (Xu *et al.* 2008; Greber, Lehrach and Adjaye 2007; Peerani *et al.* 2007; Xu *et al.* 2005). FGF2 signaling has also been implicated in neuroectoderm formation from hESCs, although its effect remains controversial (Vallier *et al.* 2009; Greber, Lehrach and Adjaye 2008; Greber *et al.* 2010; LaVaute *et al.* 2009). In particular, FGF signaling has been implicated in promoting neural induction and FGF2 is frequently used to promote self-renewal of neural stem and precursor cells. Consequently, protocols describing their derivation from ES cells incorporate the addition of FGF2 at some point (Zhang *et al*. 2001; Koch *et al.* 2009; Conti *et al.* 2005; Gerrard, Rodgers and Cui 2005). For example, WO 2008/056166 describes the differentiation of neuroectoderm progenitor cells comprising the addition of FGF2 as a differentiation factor.

For any routine application, protocols for the generation of neuroectodermal precursor cells need to be efficient and, in particular, rapid. Moreover, resulting cells must be devoid of any undifferentiated pluripotent cells, due to the ability of the latter to form tumours (teratomas) following possible cell transplantation approaches. Thus, despite the fact that a lot of effort has been invested into identifying means and methods of generating neuroectoderm cells, there is still a need for improvement, such as e.g. to enhance their use as precursor cells for toxicological studies and clinical applications.

This need is addressed by the provision of the embodiments characterised in the claims.

Accordingly, the present invention relates to a method for generating neuroectoderm cells, the method comprising: culturing pluripotent cells for about 2 to about 6 days, wherein the expression and/or activity of one or more proteins selected from the group consisting of fibroblast growth factor receptor (FGFR), son of sevenless (SOS), growth factor receptor-bound protein 2 (GRB2), rat sarcoma protein (RAS), rat fibrosarcoma (RAF), mitogen-activated protein kinase kinase (MEK) and mitogen-activated protein kinase (MAPK) is inhibited.

In accordance with the present invention, the term "neuroectoderm cells" relates to cells of neuroectodermal origin that are capable of differentiating into various neural cell types. Such neural cell types include for example neurons of the central and peripheral nervous systems, astrocytes and oligodendrocytes. Human neuroectoderm cells are characterized by the expression of key transcription factors such as PAX6, OTX1, OTX2, and NR2F2. (Zhang *et al.* 2010; Simeone *et al.* 1993; Rosa and Brivanlou 2011).

The term "pluripotent cells", in accordance with the present invention, relates to cells having the capacity for self-renewal, an ability to go through numerous cycles of cell division while maintaining their undifferentiated state, and the potential of differentiation, i.e. the capacity to differentiate into somatic cell types, i.e. the derivatives of the three germ layers: ectoderm, endoderm, and mesoderm.

Pluripotent cells, in accordance with the present invention, encompass stem cells, such as e.g. human embryonic stem cells, parthenogenetic stem cells, induced human pluripotent stem cells, mouse epiblast stem cells, or mouse induced epiblast-like pluripotent stem cells.

The term "embryonic stem cells", as used throughout the present invention, refers to stem cells derived from the inner cell mass of an early stage embryo known as a blastocyst. Recent advances in embryonic stem cell research have led to the possibility of creating new embryonic stem cell lines without destroying embryos, for example by using a single-cell biopsy similar to that used in preimplantation genetic diagnosis (PGD), which does not interfere with the embryo's developmental potential (Klimanskaya *et al.* (2006)). Furthermore, a large number of established human embryonic stem cell lines are available in the art (according to the U.S. National Institutes of Health, 21 lines are currently available for distribution to researchers), thus making it possible to work with embryonic stem cells without the necessity to destroy an embryo.

"Parthenogenetic stem cells" in accordance with the present invention, are embryonic stem cells isolated from the blastocyst stage of parthenogenetic embryos. Parthenogenetic embryos are produced by parthenogenetic (i.e. asexual) activation of non-fertilised oocytes.

"Induced pluripotent stem cells", also referred to as iPS cells herein, in accordance with the present invention, are pluripotent stem cells derived from a non-pluripotent cell, typically an adult somatic cell, by inducing a "forced" expression of certain genes. Induced pluripotent stem cells are identical to natural pluripotent stem cells, such as e.g. embryonic stem cells, in many respects including, for example, unlimited self-renewal *in vitro,* a normal karyotype, the expression of certain stem cell genes and proteins such as for example Oct3/4, Sox2, Nanog, alkaline phosphatase (ALP) as well as stem cell-specific antigen 3 and 4 (SSEA3/4), chromatin methylation patterns, doubling time, embryoid body formation, teratoma formation, viable chimera formation, and potency and differentiability (Takahashi and Yamanaka 2006, Cell 126: 663 - 676; Hanna, J., et al. (2007). Science 318(5858): 1920-3; Meissner, A., et al. (2007). Nat Biotechnol 25(10): 1177-81; Nakagawa, M., et al. (2007). Nat Biotechnol.; Okita, K., et al. (2007). Nature 448(7151): 313-7; Takahashi, K., et al. (2007Cell 131(5): 861-72; Wernig, M., et al. (2007). Nature 448(7151): 318-24; Yu, J., et al. (2007). Science 318(5858): 1917-20; Park, I. H., et al. (2008). Nature 451(7175): 141-6). Induced pluripotent stem cells are an important advancement in stem cell research, as they allow researchers to obtain pluripotent stem cells without the use of embryos (Nishikawa *et al.,* 2008). The induced pluripotent stem cells may be obtained from any adult somatic cell, preferably from fibroblasts, such as for example from skin tissue biopsies. The pluripotency of iPS cells can tested, e.g., by *in vitro* differentiation into neural, glia and cardiac cells and the production of germline chimaeric animals through blastocyst injection. Human iPS cells lines can be analysed through *in vitro* differentiation into neural, glia and cardiac cells and their *in vivo* differentiation capacity can be tested by injection into immuno-deficient SCID mice and the characterisation of resulting tumours as teratomas.

Methods for the generation of human induced pluripotent stem cells are well known to the skilled person. For example, induced pluripotent stem cells can be generated from human skin tissue biopsies (Park and Daley, 2009; Park et al., 2008). Fibroblasts are grown in MEM-medium containing chemically defined and recombinant serum components. For reprogramming, the human fibroblasts are retrovirally transduced with OCT4, SOX2, c-MYC and NANOG genes. For this, genes are cloned into a retroviral vector and transgene-expressing viral particles are produced in the HEK293FT cell line. Human skin fibroblasts are co-transduced with all four vectors. The obtained iPS cells are cultured according to protocols established for human embryonic stem cells in DMEM-medium containing serum replacement factors and recombinant growth factors. The iPS cells are analyzed for normal morphology and normal karyotype and are studied by fingerprinting analysis and immunostaining for OCT3/4, NANOG, SSEA-3, SSEA-4, Tra-1-60 and Tra-1-81. Gene transcripts for OCT4, SOX2, NANOG, KLF4, c-MYC, REX1, GDF3 and hTERT are analyzed by real-time RT-PCR. Furthermore, multilineage differentiation of iPS cells is confirmed by embryoid body, teratoma formation and differentiation into adult cell types (Choi et al., 2009; Zhang et al., 2009). As another example, human iPS cells can also be obtained from embryonic fibroblasts without viral integration using adenoviral vectors expressing c-Myc, Klf4, Oct4, and Sox2 (Zhou and Freed, 2009). Further methods are described e.g. in WO2009115295, WO2009144008 or EP2218778.

"Mouse epiblast stem cells" are derived from postimplantation epiblast tissue before gastrulation, unlike mouse embryonic stem cells,. They are maintained in a similar manner as human embryonic stem cells and respond to signaling stimuli/differentiation cues in a similar way. Hence, they are considered the counterpart of human ES cells in the mouse (Tesar et al., Nature 448,196-199, 2007). Alternatively, epiblast stem cells may be derived from pre-implantation blastocysts by applying human ES cell/epiblast stem cell culture conditions (Najm et al., Cell Stem Cell 8, 318-32, 2011). "Mouse induced epiblast-like pluripotent cells" may be obtained using direct reprogramming of somatic cells together with stringent epiblast stem cell conditions (Han et al., Nat Cell Biol. 13, 66-71, 2011).

The pluripotent cells of the invention may be obtained from a subject or derived from a cell line. The term "subject" as used herein means a vertebrate, preferably a mammal, more preferably any one of cat, dog, horse, cattle, swine, goat, sheep, mouse, rat, monkey, ape and human. In a preferred embodiment, the pluripotent cells are human pluripotent cells. More preferably, the human pluripotent cells are human embryonic stem cells or human induced pluripotent stem cells. It is preferred that the human pluripotent cells or human embryonic stem cells are obtained by methods that do not involve the destruction of a human embryo, such as for example the above described methods of using a single-cell biopsy or by employing an established cell line.

The method of the invention is carried out under suitable cell culture conditions. It is of note that general cell culture conditions are well known in the art (e.g. Cooper GM (2000). "Tools of Cell Biology", ISBN 0-87893-106-6; K. Turksen, ed., Humana Press, 2004, J. Masters, ed., Oxford University Press, 2000, "Animal cell culture", ISBN-10 0-19-963796-2). Suitable culture conditions are for example shown in more detail in the Examples of the invention.

The term "for about [...] days", as used herein, encompasses the explicitly recited amount of days as well as deviations therefrom of several hours or minutes. In other words, the time of treatment does not have to be exactly the recited amount of days but may differ by several minutes, such as for example 1 minute, 2 minutes, 3 minutes, 4 minutes, 5 minutes, 6 minutes, 7 minutes, 8 minutes, 9 minutes, 10 minutes etc. up to 59 minutes. Further, the time of treatment may differ by several hours from the recited amount of days, such as for example 1 hour, 2 hours, 3 hours, 4 hours, 5 hours, 6 hours, 7 hours, 8 hours, 9 hours, 10 hours, 11 hours, 12 hours etc.. As an example, the term "about 2 days" encompasses exactly two days, i.e. 48 hours, but also encompasses for example 36 hours, 37 hours, 38 hours, 39 hours, 40 hours, 41 hours, 42 hours, 43 hours, 44 hours, 45 hours, 46 hours, 47 hours, 49 hours, 50 hours, 51 hours, 52 hours, 53 hours, 54 hours, 55 hours, 56 hours, 57 hours, 58 hours, 59 hours, 60 hours etc. The skilled person is aware that such time spans of treatment are relative amounts of time that do not require a 100% accuracy as long as the time span approximately corresponds to the recited amount. Accordingly, a deviation from the recited value of for example 15%, more preferably of 10%, and most preferably of 5% is encompassed by the term "about".

The term "wherein the expression and/or activity of [...] is inhibited", in accordance with the present invention, relates to a reduction or abolishment in expression or activity of the recited molecule. Said reduction may be achieved by one of the following effects: (i) the transcription of the gene encoding the protein to be inhibited is reduced or abolished, (ii) the translation of the mRNA encoding the protein to be inhibited is reduced or abolished, (iii) the protein performs its biochemical function with reduced or abolished efficiency, and (iv) the protein performs its cellular function with reduced or abolished efficiency.

Compounds suitable to achieve the effect described in (i) include compounds interfering with the transcriptional machinery and/or its interaction with the promoter of said gene and/or with expression control elements remote from the promoter such as enhancers. Compounds suitable to achieve the effect described in (ii) comprise antisense constructs and constructs for performing RNA interference (e.g. siRNA, shRNA, miRNA) well known in the art (see, e.g. Zamore (2001) Nat. Struct. Biol. 8(9), 746; Tuschl (2001) Chembiochem. 2(4), 239). Compounds suitable to achieve the effect described in (iii) interfere with molecular functions of the protein to be inhibited. Compounds suitable to achieve the effect described in (iv) include compounds which do not necessarily bind directly to the target protein, but still interfere with their activity, for example by binding to and/or inhibiting the function or expression of members of a pathway which comprises the target protein. These members may be either upstream or downstream of the protein to be inhibited within said pathway.

Such compounds further include, without being limiting, antibodies, aptamers, ribozymes and small molecules. Suitable compounds further include but are not limited to, for example, peptides such as soluble peptides, including Ig-tailed fusion peptides and members of random peptide libraries (see, e.g., Lam et al. (1991) Nature 354: 82-84; Houghten et al. (1991) Nature 354: 84-86) and combinatorial chemistry-derived molecular libraries made of D-and/or L-configuration amino acids or phosphopeptides (e.g., members of random and partially degenerate, directed phosphopeptide libraries, see, e.g., Songyang et al. (1993) Cell 72: 767-778).

The term "antisense nucleic acid molecule" is known in the art and refers to a nucleic acid which is complementary to a target nucleic acid. An antisense molecule in accordance with the invention is capable of interacting with the target nucleic acid, more specifically it is capable of hybridising with the target nucleic acid. Due to the formation of the hybrid, transcription of the target gene(s) and/or translation of the target mRNA is reduced or blocked. Standard methods relating to antisense technology have been described (see, e.g., Melani et al., Cancer Res. (1991) 51:2897-2901).

In accordance with the present invention, the term "small interfering RNA (siRNA)", also known as short interfering RNA or silencing RNA, refers to a class of 18 to 30, preferably 19 to 25, most preferred 21 to 23 or even more preferably 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome.

siRNAs naturally found in nature have a well defined structure: a short double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Essentially any gene of which the sequence is known can thus be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Exogenously introduced siRNAs may be devoid of overhangs at their 3' and 5' ends, however, it is preferred that at least one RNA strand has a 5'- and/or 3'-overhang. Preferably, one end of the double-strand has a 3'-overhang from 1-5 nucleotides, more preferably from 1-3 nucleotides and most preferably 2 nucleotides. The other end may be blunt-ended or has up to 6 nucleotides 3'-overhang. In general, any RNA molecule suitable to act as siRNA is envisioned in the present invention. The most efficient silencing was so far obtained with siRNA duplexes composed of 21-nt sense and 21-nt antisense strands, paired in a manner to have 2-nt 3' overhangs on either end. The sequence of the 2-nt 3' overhang makes a small contribution to the specificity of target recognition restricted to the unpaired nucleotide adjacent to the first base pair (Elbashir et al. 2001). 2'-deoxynucleotides in the 3' overhangs are as efficient as ribonucleotides, but are often cheaper to synthesize and probably more nuclease resistant. Delivery of siRNA may be accomplished using any of the methods known in the art, for example by combining the siRNA with saline and administering the combination intravenously or intranasally or by formulating siRNA in glucose (such as for example 5% glucose) or cationic lipids and polymers can be used for siRNA delivery *in vivo* through systemic routes either intravenously (IV) or intraperitoneally (IP) (Fougerolles et al. (2008), Current Opinion in Pharmacology, 8:280-285; Lu et al. (2008), Methods in Molecular Biology, vol. 437: Drug Delivery Systems - Chapter 3: Delivering Small Interfering RNA for Novel Therapeutics).

A short hairpin RNA (shRNA) is a sequence of RNA that makes a tight hairpin turn that can be used to typically silence gene expression via RNA interference. shRNA can for example use a vector introduced into cells, in which case the U6 promoter is utilized to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs which match the siRNA that is bound to it.

Preferably, si/shRNAs to be used in the present invention are chemically synthesized using conventional methods that, for example, appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK). Most conveniently, siRNAs or shRNAs are obtained from commercial RNA oligo synthesis suppliers, which sell RNA-synthesis products of different quality and costs. In general, the RNAs applicable in the present invention are conventionally synthesized and are readily provided in a quality suitable for RNAi.

Further molecules effecting RNAi include, for example, microRNAs (miRNA). Said RNA species are single-stranded RNA molecules which, as endogenous RNA molecules, regulate gene expression. Binding to a complementary mRNA transcript triggers the degradation of said mRNA transcript through a process similar to RNA interference. Accordingly, miRNA may be employed as an inhibitor of the of the biomarkers in accordance with the present invention.

The term "antibody" as used in accordance with the present invention comprises polyclonal and monoclonal antibodies, as well as derivatives or fragments thereof, which still retain the binding specificity. Antibody fragments or derivatives comprise, inter alia, Fab or Fab' fragments as well as Fd, F(ab')₂, Fv or scFv fragments; see, for example Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. The term "antibody" also includes embodiments such as chimeric (human constant domain, non-human variable domain), single chain and humanised (human antibody with the exception of non-human CDRs) antibodies.

Various techniques for the production of antibodies are well known in the art and described, e.g. in Harlow and Lane (1988) and (1999), loc. cit. Thus, the antibodies can be produced by peptidomimetics. Further, techniques described for the production of single chain antibodies (see, inter alia, US Patent 4,946,778) can be adapted to produce single chain antibodies specific for the target of this invention. Also, transgenic animals or plants (see, e.g., US patent 6,080,560) may be used to express (humanized) antibodies specific for the target of this invention. Most preferably, the antibody is a monoclonal antibody, such as a human or humanized antibody. For the preparation of monoclonal antibodies, any technique which provides antibodies produced by continuous cell line cultures can be used. Examples for such techniques are described, e.g. in Harlow and Lane (1988) and (1999), loc. cit. and include the hybridoma technique (Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BlAcore system can be used to increase the efficiency of phage antibodies which bind to an epitope of STIM2 or an epitope of a STIM2-regulated plasma membrane calcium channel (Schier, Human Antibodies Hybridomas 7 (1996), 97-105; Malmborg, J. Immunol. Methods 183 (1995), 7-13). It is also envisaged in the context of this invention that the term "antibody" comprises antibody constructs which may be expressed in cells, e.g. antibody constructs which may be transfected and/or transduced via, inter alia, viruses or plasmid vectors.

Aptamers are nucleic acid molecules or peptide molecules that bind a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications (Osborne et. al. (1997), Current Opinion in Chemical Biology, 1:5-9; Stull & Szoka (1995), Pharmaceutical Research, 12, 4:465-483).

More specifically, aptamers can be classified as nucleic acid aptamers, such as DNA or RNA aptamers, or peptide aptamers. Whereas the former normally consist of (usually short) strands of oligonucleotides, the latter preferably consist of a short variable peptide domain, attached at both ends to a protein scaffold.

Nucleic acid aptamers are nucleic acid species that, as a rule, have been engineered through repeated rounds of *in vitro* selection or equivalently, SELEX (systematic evolution of ligands by exponential enrichment) to bind to various molecular targets such as small molecules, proteins, nucleic acids, and even cells, tissues and organisms.

Peptide aptamers usually are peptides or proteins that are designed to interfere with other protein interactions inside cells. They typically consist of a variable peptide loop attached at both ends to a protein scaffold. This double structural constraint greatly increases the binding affinity of the peptide aptamer to levels comparable to an antibody's (nanomolar range). The variable peptide loop typically comprises 10 to 20 amino acids, and the scaffold may be any protein having good solubility properties. Currently, the bacterial protein Thioredoxin-A is the most commonly used scaffold protein, the variable peptide loop being inserted within the redox-active site, which is a -Cys-Gly-Pro-Cys- loop in the wild type protein, the two cysteins lateral chains being able to form a disulfide bridge. Peptide aptamer selection can be made using different systems, but the most widely used is currently the yeast two-hybrid system.

Aptamers offer the utility for biotechnological and therapeutic applications as they offer molecular recognition properties that rival those of the commonly used biomolecules, in particular antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications. Non-modified aptamers are cleared rapidly from the bloodstream, with a half-life of minutes to hours, mainly due to nuclease degradation and clearance from the body by the kidneys, a result of the aptamer's inherently low molecular weight. Unmodified aptamer applications currently focus on treating transient conditions such as blood clotting, or treating organs such as the eye where local delivery is possible. This rapid clearance can be an advantage in applications such as *in vivo* diagnostic imaging. Several modifications, such as 2'-fluorine-substituted pyrimidines, polyethylene glycol (PEG) linkage, fusion to albumin or other half life extending proteins etc. are available to scientists such that the half-life of aptamers can be increased for several days or even weeks.

The term "peptide" as used herein describes a group of molecules consisting of up to 30 amino acids, whereas the term "protein" as used herein describes a group of molecules consisting of more than 30 amino acids. Peptides and proteins may further form dimers, trimers and higher oligomers, i.e. consisting of more than one molecule which may be identical or non-identical. The corresponding higher order structures are, consequently, termed homo- or heterodimers, homo- or heterotrimers etc. The terms "peptide" and "protein" (wherein "protein" is interchangeably used with "polypeptide") also refer to naturally modified peptides/proteins wherein the modification is effected e.g. by glycosylation, acetylation, phosphorylation and the like. Such modifications are well-known in the art.

A ribozyme (from ribonucleic acid enzyme, also called RNA enzyme or catalytic RNA) is an RNA molecule that catalyzes a chemical reaction. Many natural ribozymes catalyze either their own cleavage or the cleavage of other RNAs, but they have also been found to catalyze the aminotransferase activity of the ribosome. Non-limiting examples of well-characterized small self-cleaving RNAs are the hammerhead, hairpin, hepatitis delta virus, and in vitro-selected lead-dependent ribozymes, whereas the group I intron is an example for larger ribozymes. The principle of catalytic self-cleavage has become well established in the last 10 years. The hammerhead ribozymes are characterized best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site. The basic principle of constructing hammerhead ribozymes is as follows: An interesting region of the RNA, which contains the GUC (or CUC) triplet, is selected. Two oligonucleotide strands, each usually with 6 to 8 nucleotides, are taken and the catalytic hammerhead sequence is inserted between them. Molecules of this type were synthesized for numerous target sequences. They showed catalytic activity *in vitro* and in some cases also *in vivo.* The best results are usually obtained with short ribozymes and target sequences.

A recent development, also useful in accordance with the present invention, is the combination of an aptamer recognizing a small compound with a hammerhead ribozyme. The conformational change induced in the aptamer upon binding the target molecule is supposed to regulate the catalytic function of the ribozyme.

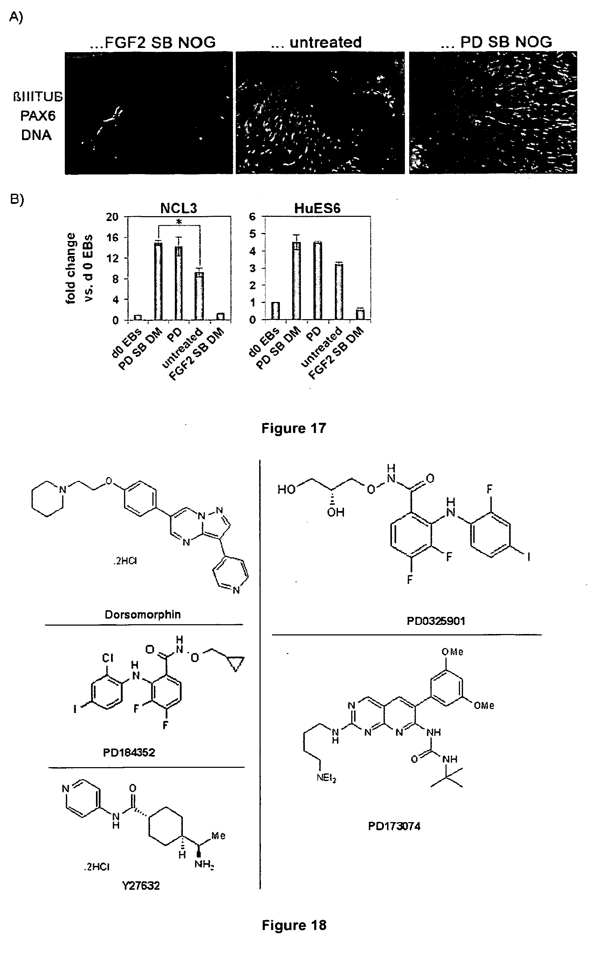

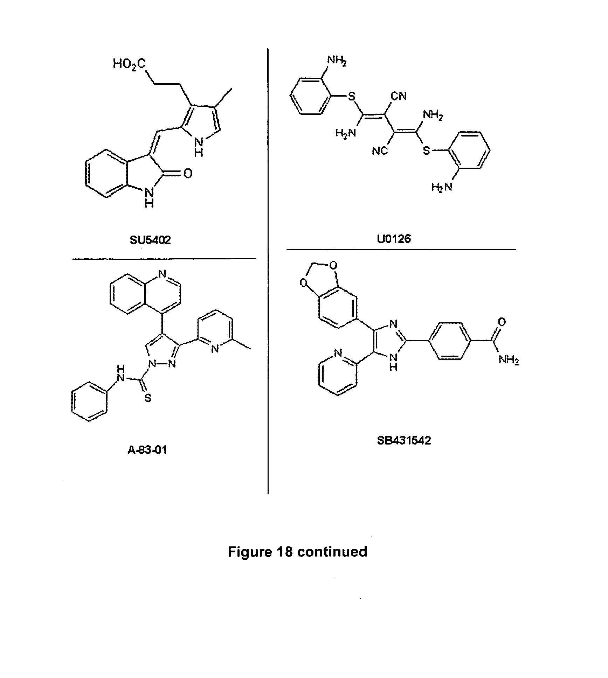

A "small molecule" according to the present invention may be, for example, an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. Alternatively, the "small molecule" in accordance with the present invention may be an inorganic compound. Inorganic compounds are derived from mineral sources and include all compounds without carbon atoms (except carbon dioxide, carbon monoxide and carbonates). Preferably, the small molecule has a molecular weight of less than about 2000 amu, or less than about 1000 amu such as less than about 500 amu, and even more preferably less than about 250 amu. The size of a small molecule can be determined by methods well-known in the art, e.g., mass spectrometry. The small molecules may be designed, for example, based on the crystal structure of the target molecule, where sites presumably responsible for the biological activity, can be identified and verified in *in vivo* assays such as *in vivo* high-throughput screening (HTS) assays. Such small molecules may be particularly suitable to inhibit protein-protein-interaction by blocking specific bindings sites of the target molecule. Suitable small molecules currently employed in the inhibition of the recited proteins include, without being limiting: 4-[4-(3,4-Methylenedioxyphenyl)-5-(2-pyridyl)-1 H-imidazol-2-yl]-benzamide hydrate (SB431542), 3-(6-Methylpyridin-2-yl)-1-phenylthiocarbamoyl-4-quinolin-4-ylpyrazole (A-83-01), 6-[4-[2-(1-Piperidinyl)ethoxy]phenyl]-3-(4-pyridinyl)-pyrazolo[1,5-a]pyrimidine dihydrochloride (dorsomorphin), 2-(2-Chloro-4-iodo-phenylamino)-N-cyclopropylmethoxy-3,4-difluoro-benzamide (PD184352), N-[(2R)-2,3-dihydroxypropoxy]-3,4-difluoro-2-[(2-fluoro-4-iodophenyl)amino]-benzamide (PD0325901 2'-Amino-3'-methoxyflavone (PD98059) and 1,4-diamino-2,3-dicyano-1,4-bis (2-aminophenylthio)butadiene (U0126); and/or wherein the activity of FGFR is inhibited by 2-[(1,2-Dihydro-2-oxo-3H-indol-3-ylidene)methyl]-4-methyl-1H-pyrrole-3-propanoic acid (SU5402) and/or N-[2-[[4-(Diethylamino)butyl]amino]-6-(3,5-dimethoxyphenyl)pyrido[2,3-d]pyrimidin-7-yl]-N'-(1,1-dimethylethyl)urea (PD173074). These molecules are well known in the art and are shown, for example, in Figure 18.

Also encompassed herein are modified versions of these inhibitory compounds.

The term "modified versions of these inhibitory compounds" in accordance with the present invention refers to versions of the compounds that are modified to achieve i) modified spectrum of activity, organ specificity, and/or ii) improved potency, and/or iii) decreased toxicity (improved therapeutic index), and/or iv) decreased side effects, and/or v) modified onset of therapeutic action, duration of effect, and/or vi) modified pharmacokinetic parameters (resorption, distribution, metabolism and excretion), and/or vii) modified physico-chemical parameters (solubility, hygroscopicity, color, taste, odor, stability, state), and/or viii) improved general specificity, organ/tissue specificity, and/or ix) optimised application form and route by, for example, (a) esterification of carboxyl groups, or (b) esterification of hydroxyl groups with carboxylic acids, or (c) esterification of hydroxyl groups to, e.g. phosphates, pyrophosphates or sulfates or hemi-succinates, or (d) formation of pharmaceutically acceptable salts, or (e) formation of pharmaceutically acceptable complexes, or (f) synthesis of pharmacologically active polymers, or (g) introduction of hydrophilic moieties, or (h) introduction/exchange of substituents on aromates or side chains, change of substituent pattern, or (i) modification by introduction of isosteric or bioisosteric moieties, or (j) synthesis of homologous compounds, or (k) introduction of branched side chains, or (k) conversion of alkyl substituents to cyclic analogues, or (I) derivatisation of hydroxyl groups to ketales, acetales, or (m) N-acetylation to amides, phenylcarbamates, or (n) synthesis of Mannich bases, imines, or (o) transformation of ketones or aldehydes to Schiffs bases, oximes, acetales, ketales, enolesters, oxazolidines, thiazolidines; or combinations thereof.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

In a preferred embodiment, the expression and/or activity is reduced such that it is less than 90%, more preferred less than 80%, less than 70%, less than 60% or less than 50% of the expression and/or activity in the absence of any inhibition. Even more preferred is that the expression and/or activity is reduced such that it is less than 25%, more preferred less than 10%, less than 5%, or less than 1% of the expression and/or activity in the absence of any inhibition. Most preferably, the expression and/or activity of the target protein is fully inhibited, i.e. no expression or activity is detectable.

The efficiency of an inhibitor can be quantified by comparing the level of expression and/or activity in the presence of an inhibitor to that in the absence of the inhibitor. For example, as a measure may be used: the change in amount of mRNA formed, the change in amount of protein formed, the change in biological activity of the target proteins as described herein below, and/or the change in the cellular phenotype or in the phenotype of an organism. In other words, the efficiency of an inhibitor can be quantified by comparing e.g. the amount of target protein in the presence of an inhibitor to that in the absence of the inhibitor or by determining the biological activity of the target protein present prior to and after administration of the inhibitor, wherein a reduction in the amount or biological activity of the target protein in the presence of or after administration of the inhibitor as compared to in the absence of or prior to said administration is indicative of a successful inhibition of the target protein. Means and methods to determine the amount of mRNA or proteins in a sample or for determining biological activities are well known in the art and include, without being limiting, the following methods.

The determination of binding of potential inhibitors can be effected in, for example, any binding assay, preferably biophysical binding assay, which may be used to identify binding test molecules prior to performing the functional/activity assay with the inhibitor. Suitable biophysical binding assays are known in the art and comprise fluorescence polarisation (FP) assay, fluorescence resonance energy transfer (FRET) assay and surface plasmon resonance (SPR) assay. For example, a modulator acting via binding to an enzyme, and thereby modulating the activity of said enzyme, may be tested by FRET by labelling either the modulator or the enzyme with a donor chromophore and the other molecule with an acceptor chromophore. These chromophore-labelled molecules are then mixed with each other. When they are dissociated, donor emission can be detected upon donor excitation at the appropriate wavelength. However, when the donor and acceptor are in proximity (1-10 nm) due to the interaction of the modulator with the enzyme, the acceptor emission is predominantly observed because of the intermolecular FRET from the donor to the acceptor.

In cases where an inhibitor acts by affecting the expression level of the target protein, the determination of the expression level of the protein can, for example, be carried out on the nucleic acid level or on the amino acid level.

Methods for determining the expression of a protein on the nucleic acid level include, but are not limited to, northern blotting, PCR, RT-PCR or real RT-PCR. PCR is well known in the art and is employed to make large numbers of copies of a target sequence. This is done on an automated cycler device, which can heat and cool containers with the reaction mixture in a very short time. The PCR, generally, consists of many repetitions of a cycle which consists of: (a) a denaturing step, which melts both strands of a DNA molecule and terminates all previous enzymatic reactions; (b) an annealing step, which is aimed at allowing the primers to anneal specifically to the melted strands of the DNA molecule; and (c) an extension step, which elongates the annealed primers by using the information provided by the template strand. Generally, PCR can be performed, for example, in a 50 µl reaction mixture containing 5 µl of 10 x PCR buffer with 1.5 mM MgCl₂, 200 µM of each deoxynucleoside triphosphate, 0.5 µl of each primer (10 µM), about 10 to 100ng of template DNA and 1 to 2.5 units of Taq Polymerase. The primers for the amplification may be labeled or be unlabeled. DNA amplification can be performed, e.g., with a model 2400 thermal cycler (Applied Biosystems, Foster City, CA): 2 min at 94°C, followed by 30 to 40 cycles consisting of annealing (e. g. 30 s at 50°C), extension (e. g. 1 min at 72°C, depending on the length of DNA template and the enzyme used), denaturing (e. g. 10 s at 94°C) and a final annealing step at 55°C for 1 min as well as a final extension step at 72°C for 5 min. Suitable polymerases for use with a DNA template include, for example, E. coli DNA polymerase I or its Klenow fragment, T4 DNA polymerase, Tth polymerase, Taq polymerase, a heat-stable DNA polymerase isolated from Thermus aquaticus Vent, Amplitaq, Pfu and KOD, some of which may exhibit proof-reading function and/or different temperature optima. However, it is well known in the art how to optimize PCR conditions for the amplification of specific nucleic acid molecules with primers of different length and/or composition or to scale down or increase the volume of the reaction mix. The "reverse transcriptase polymerase chain reaction" (RT-PCR) is used when the nucleic acid to be amplified consists of RNA. The term "reverse transcriptase" refers to an enzyme that catalyzes the polymerization of deoxyribonucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerizing agents that convert the RNA target sequence into a complementary, copy-DNA (cDNA) sequence are avian myeloblastosis virus reverse transcriptase and Thermus thermophilus DNA polymerase, a thermostable DNA polymerase with reverse transcriptase activity marketed by Perkin Elmer. Typically, the genomic RNA/cDNA duplex template is heat denatured during the first denaturation step after the initial reverse transcription step leaving the DNA strand available as an amplification template. High-temperature RT provides greater primer specificity and improved efficiency. U.S. patent application Serial No. 07/746, 121, filed Aug. 15, 1991, describes a "homogeneous RT-PCR" in which the same primers and polymerase suffice for both the reverse transcription and the PCR amplification steps, and the reaction conditions are optimized so that both reactions occur without a change of reagents. Thermus thermophilus DNA polymerase, a thermostable DNA polymerase that can function as a reverse transcriptase, can be used for all primer extension steps, regardless of template. Both processes can be done without having to open the tube to change or add reagents; only the temperature profile is adjusted between the first cycle (RNA template) and the rest of the amplification cycles (DNA template). The RT Reaction can be performed, for example, in a 20µl reaction mix containing: 4 µl of 5x AMV-RT buffer, 2 µl of Oligo dT (100 µg/ml), 2µl of 10 mM dNTPs, 1µl total RNA, 10 Units of AMV reverse transcriptase, and H₂O to 20µl final volume. The reaction may be, for example, performed by using the following conditions: The reaction is held at 70 C° for 15 minutes to allow for reverse transcription. The reaction temperature is then raised to 95 C° for 1 minute to denature the RNA-cDNA duplex. Next, the reaction temperature undergoes two cycles of 95°C for 15 seconds and 60 C° for 20 seconds followed by 38 cycles of 90 C° for 15 seconds and 60 C° for 20 seconds. Finally, the reaction temperature is held at 60 C° for 4 minutes for the final extension step, cooled to 15 C°, and held at that temperature until further processing of the amplified sample. Any of the above mentioned reaction conditions may be scaled up according to the needs of the particular case. The resulting products are loaded onto an agarose gel and band intensities are compared after staining the nucleic acid molecules with an intercalating dye such as ethidiumbromide or SybrGreen. A lower band intensity of the sample treated with the inhibitor as compared to a non-treated sample indicates that the inhibitor successfully inhibits the expression of the protein on the nucleic acid level.

Real-time PCR employs a specific probe, in the art also referred to as TaqMan probe, which has a reporter dye covalently attached at the 5' end and a quencher at the 3' end. After the TaqMan probe has been hybridized in the annealing step of the PCR reaction to the complementary site of the polynucleotide being amplified, the 5' fluorophore is cleaved by the 5' nuclease activity of Taq polymerase in the extension phase of the PCR reaction. This enhances the fluorescence of the 5' donor, which was formerly quenched due to the close proximity to the 3' acceptor in the TaqMan probe sequence. Thereby, the process of amplification can be monitored directly and in real time, which permits a significantly more precise determination of expression levels than conventional end-point PCR. Also of use in Real-time RT-PCR experiments is a DNA intercalating dye such as SybrGreen for monitoring the de novo synthesis of double stranded DNA molecules.

Methods for the determination of the expression of a protein on the amino acid level include but are not limited to western blotting or polyacrylamide gel electrophoresis in conjunction with protein staining techniques such as Coomassie Brilliant blue or silver-staining. The total protein is loaded onto a polyacrylamide gel and electrophoresed. Afterwards, the separated proteins are transferred onto a membrane, e.g. a polyvinyldifluoride (PVDF) membrane, by applying an electrical current. The proteins on the membrane are exposed to an antibody specifically recognising the protein of interest. After washing, a second antibody specifically recognising the first antibody and carrying a readout system such as a fluorescent dye is applied. The amount of the protein of interest is determined by comparing the fluorescence intensity of the protein derived from the sample treated with the inhibitor and the protein derived from a non-treated sample. A lower fluorescence intensity of the protein derived from the sample treated with the inhibitor indicates a successful inhibitor of the protein. Also of use in protein quantification is the Agilent Bioanalyzer technique.

The function of an inhibitor suitable for the method of the present invention may be identified and/or verified by using high throughput screening assays (HTS). High-throughput assays, independently of being biochemical, cellular or other assays, generally may be performed in wells of microtiter plates, wherein each plate may contain, for example 96, 384 or 1536 wells. Handling of the plates, including incubation at temperatures other than ambient temperature, and bringing into contact of test compounds with the assay mixture is preferably effected by one or more computer-controlled robotic systems including pipetting devices. Where large libraries of test compounds are to be screened and/or screening is to be effected within short time, mixtures of, for example 10, 20, 30, 40, 50 or 100 test compounds may be added to each well. In case a well exhibits biological activity, said mixture of test compounds may be de-convoluted to identify the one or more test compounds in said mixture giving rise to the observed biological activity.

In accordance with the present invention, the term "fibroblast growth factor receptor (FGFR)" refers to a receptor consisting of an extracellular ligand domain composed of three immunoglobulin-like domains, a single transmembrane helix domain, and an intracellular domain with tyrosine kinase activity, that binds fibroblast growth factors. So far, four distinct membrane FGFR have been identified in mammals: FGFR1 (also referred to as CD331), FGFR2 (also referred to as CD332), FGFR3 (also referred to as CD333) and FGFR4 (also referred to as CD334). Human FGFR1 to FGFR4 are for example represented by the Entrez Gene IDs 2260, 2263, 2261 and 2264, respectively, and the UniProt lDs P11362, P21802, P22607 and P22455, respectively.

The term "SOS", as used herein, relates to the son of sevenless homolog gene and its product. The term comprises for example SOS1 and SOS2, which are guanine-nucleotide releasing factors, i.e. they promotes the exchange of Ras-bound GDP by GTP. Human SOS1 is for example represented by Entrez Gene ID 6654 and UniProt ID Q07889 while human SOS2 is represented for example by Entrez Gene ID 6655 and UniProt ID Q07890.

As used herein, the term "GRB2" relates to the growth factor receptor-bound protein 2 gene and its product, which is an adapter protein that provides a critical link between cell surface growth factor receptors and the Ras signaling pathway. Human GRB2 is represented for example by Entrez Gene ID 2885 and UniProt ID P62993.

The term "RAS", as used herein, relates to the family of rat sarcoma genes, and their products. The family comprises for example KRAS, NRAS, and HRAS. RAS proteins bind GDP/GTP and possess intrinsic GTPase activity. Human KRAS is for example represented by Entrez Gene ID 3845 and UniProt ID P01116; human NRAS is represented for example by Entrez Gene ID 4893 and UniProt ID P01111; while human HRAS is represented for example by Entrez Gene ID 3265 and UniProt ID P01112.

The term "RAF", as used herein, relates to the proto-oncogene serine/threonine-protein kinase and its product RAF1. RAF is involved in the transduction of mitogenic signals from the cell membrane to the nucleus and is part of the Ras-dependent signaling pathway from receptors to the nucleus. Human RAF1 is for example represented by Entrez Gene ID 5894 and UniProt ID P04049.

The term "MEK", as used herein, relates to the family of mitogen-activated protein kinase kinases. This family comprises for example the genes MAP2K1, and MAP2K2 and their products. Mitogen-activated protein kinase kinases catalyse the concomitant phosphorylation of a threonine and a tyrosine residue in a Thr-Glu-Tyr sequence located in MAP kinases and activate ERK1 and ERK2 MAP kinases. Human MAP2KI is represented for example by Entrez Gene ID 5604 and UniProt ID Q02750 while human MAP2K2 is for example represented by Entrez Gene ID 5605 and UniProt ID P36507.

The terms "MAPK" or "ERK", as used herein, relate to the family of mitogen-activated protein kinases. This family comprises for example the genes MAPK1 and MAPK3 and their products. Mitogen-activated protein kinases phosphorylate a number of transcription factors such as ELK1. They also phosphorylate EIF4EBP1, microtubule-associated protein 2 (MAP2) and SPZ1. MAPK1 further acts as a transcriptional repressor, wherein the transcriptional activity is independent of kinase activity. Human MAPK1 is represented for example by Entrez Gene ID 5594 and UniProt ID P28482 while human MAPK2 is for example represented by Entrez Gene ID 5595 and UniProt ID P27361.

As used herein, the term "one or more proteins selected from [...]" relates to at least one protein selected from the recited list. Thus, the term encompasses one protein or two proteins or three proteins or four proteins or five proteins or six proteins or seven proteins, wherein these proteins are selected from the recited list. Also encompassed by the method of the present invention is that additional proteins are inhibited, wherein said additional proteins may or may not be selected from the recited list. In other words, whereas at least "one or more proteins" has to be chosen from fibroblast growth factor receptor (FGFR), son of sevenless (SOS), growth factor receptor-bound protein 2 (GRB2), rat sarcoma protein (RAS), rat fibrosarcoma (RAF), mitogen-activated protein kinase kinase (MEK) and mitogen-activated protein kinase (MAPK), further different proteins may additionally be inhibited in accordance with the present invention. Inhibition of additional proteins may for example be effected by addition of ROCK inhibitor Y27632 (to inhibit Rho-associated coiled-coil containing kinase: ROCK1 and ROCK2, Watanabe et al., Nat. Biotech. 25, 681-686, 2007), either for the entire time of cell culture in accordance with the method of the present invention or for restricted time periods during said cell culture, such as for example for the first 3 days of culture.

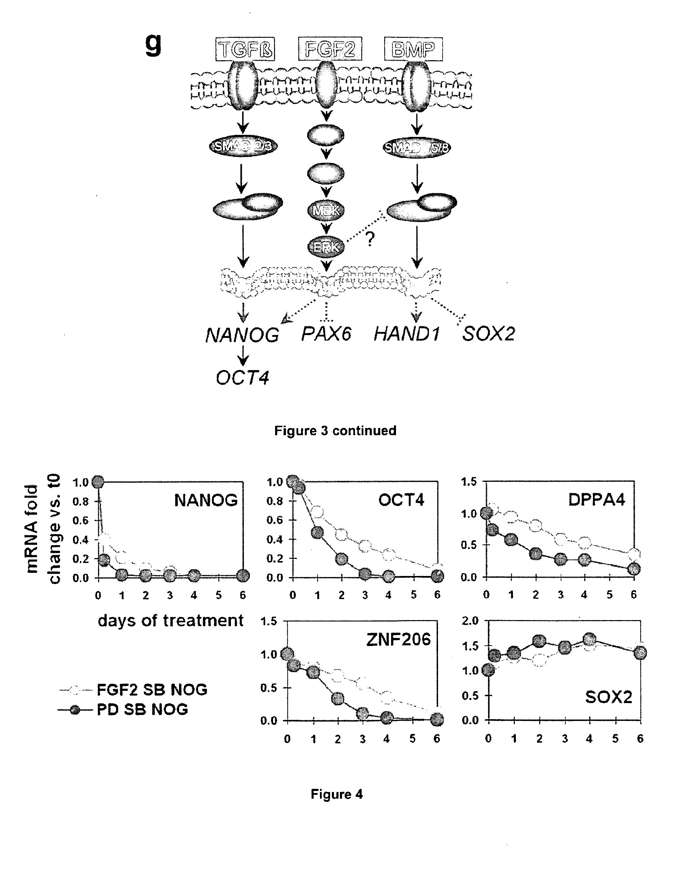

In accordance with the present invention, a novel role for FGF2 signaling in hESCs was surprisingly discovered (Fig. 3g herein), namely to prevent neuroectoderm induction by repressing *PAX6,* the key fate determinant of that lineage in human (Zhang *et a*/*.* 2010). FGF2 supplementation inhibited *PAX6* induction throughout all experiments. Furthermore, in the absence of FGF2, and in particular when FGF/ERK was inhibited, *PAX6* expression was activated, leading to the conclusion that this may be the key mechanism underlying neuroectoderm conversion from hESCs.

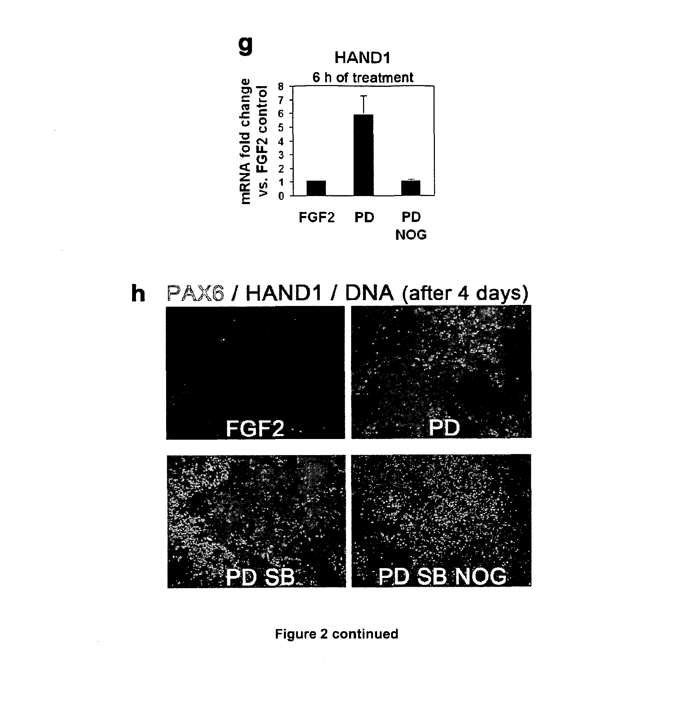

At first sight, this model appears to be at odds with published work, as FGF signaling has frequently been implicated in promoting neural induction. However, most of these data have been generated in model organisms such as chick and Xenopus (Bottcher and Niehrs 2005), yet little appears to be known about pre-gastrulation stage mammalian embryos. Moreover, FGF2 is frequently used to promote self-renewal of neural stem and precursor cells, and protocols describing their derivation from ES cells incorporate the addition of FGF2 at some point (Zhang *et al.* 2001; Koch *et al.* 2009; Conti *et al.* 2005; Gerrard, Rodgers and Cui 2005). Nonetheless, the present model suggests that FGF2 inhibits the initial induction of *PAX6.* It was also observed that when hESCs are grown in defined FGF2 media, larger hESC colonies usually display some PAX6⁺ cells in their centres (see e.g. Fig. 2h, left panel). Hence, FGF2 signaling apparently does not fully block but inhibits *PAX6* induction in hESCs.

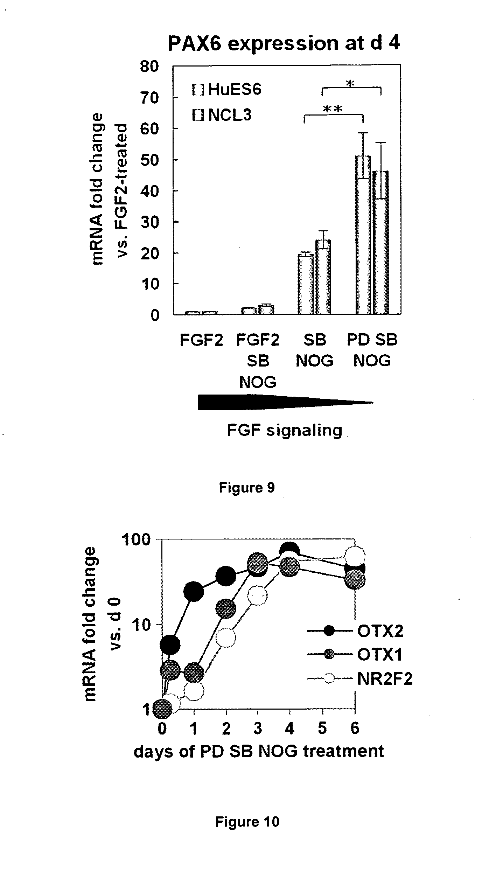

As shown for example in Figure 9, the neuroectodermal induction method of the present invention resulted in an up-regulation of PAX6 expression that was approximately twice as high as that observed in the presence of TGFβ/BMP inhibitors alone, as previously described in the art (e.g. Chambers *et al.* 2009⁹). Thus, it was surprisingly found that inhibition of the FGF2 signalling pathway is particularly effective in promoting differentiation of hES cells.

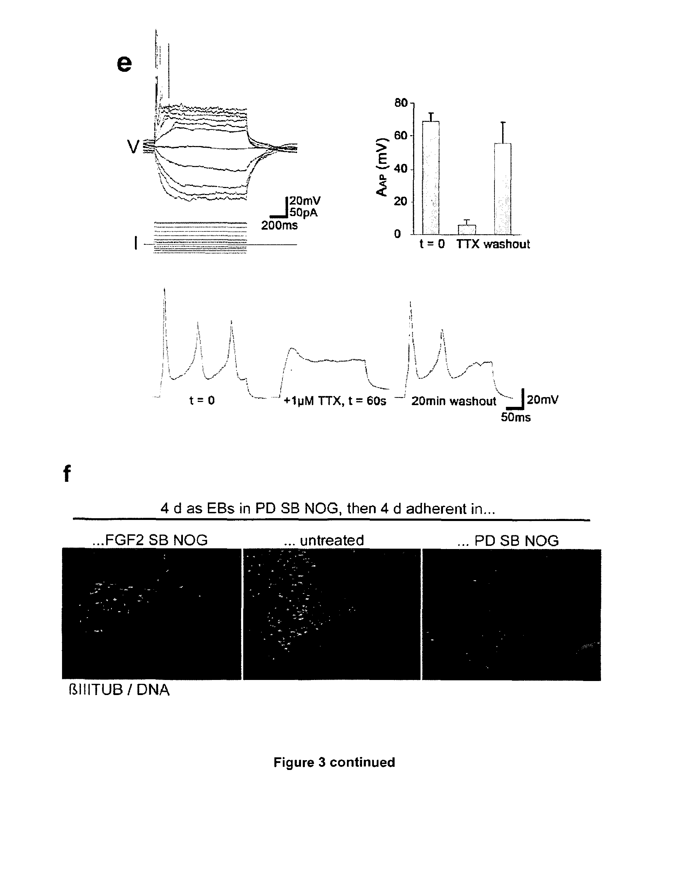

Based on the surprising findings of the present invention, a novel method for generating neuroectoderm cells has been developed. These cells that can rapidly give rise to functional neurons at high efficiency, implying that ERK signaling is dispensable in this entire differentiation process. Without wishing to be bound by any theory, the present inventors stipulate that this may not only be due to a failure in up-regulation of *PAX6* expression during the embryoid body phase, but that FGF2/ERK signaling also inhibits differentiation at a later stage. In fact, this hypothesis appears quite likely, as hESC-derived neural precursor cells can readily self-renew in FGF2-containing media (Zhang *et al.* 2001; Koch *et al.* 2009). As shown in the appended examples, it was confirmed that FGF2 also plays an inhibitory role in the second phase of differentiation (Fig. 3f).

In a preferred embodiment of the method of the invention, only proteins from the recited list of fibroblast growth factor receptor (FGFR), son of sevenless (SOS), growth factor receptor-bound protein 2 (GRB2), rat sarcoma protein (RAS), rat fibrosarcoma (RAF), mitogen-activated protein kinase kinase (MEK) and mitogen-activated protein kinase (MAPK), but no additional proteins, are inhibited.

In a preferred embodiment of the method for generating neuroectoderm cells, said human pluripotent cells are cultured for at least about 4 days.

As is shown in the experimental data below, a particularly pronounced PAX6 expression was obtained in most cells after four days of culture in accordance with the method of the invention. The neuroectodermal identity of this population was confirmed by the induction of other markers, such as *OTX1* and *OTX2,* which were also expressed after four days in culture.

In another preferred embodiment of the method of the invention, additionally the nodal/activin/tumour growth factor beta/small mothers against decapentaplegic 2/3 (Nodal/activin/TGFbeta/SMAD2/3) signalling pathway; and/or the bone morphogenic protein/growth differentiation factor/small mothers against decapentaplegic 1/5/8 (BMP/GDF/SMAD1/5/8) signalling pathway is inhibited.

The "nodal/activin/tumour growth factor beta/small mothers against decapentaplegic 2/3 (Nodal/activin[TGFbeta/SMAD2/3) signalling pathway" is well known in the art and has been described, for example, in Feng and Derynck 2005.

The "bone morphogenic protein/growth differentiation factor/small mothers against decapentaplegic 1/5/8 (BMP/GDF/SMAD1/5/8) signalling pathway" is well known in the art and has been described, for example, in in Feng and Derynck 2005.

With regard to the inhibition of these pathways, the definitions provided herein above with regard to inhibition of proteins apply *mutatis mutandis.* For example, the same degree of inhibition, the same effects, i.e. the effects described as (i) to (iv) above, but also the same methods for testing or identifiy potential inhibitory compounds, also apply to the inhibition of these two additional signalling pathways in accordance with this preferred embodiment.

In a more preferred embodiment of the method of the invention, the nodal/activin/TGFbeta/SMAD2/3 signalling pathway is inhibited by one or more compounds selected from the group consisting of 4-[4-(3,4-Methylenedioxyphenyl)-5-(2-pyridyl)-1 H-imidazol-2-yl]-benzamide hydrate (SB431542; Laping *et al.* 2002), 3-(6-Methylpyridin-2-yi)-l-phenylthio-carbamoyl-4-quinolin-4-ylpyrazole (A-83-01; Tojo *et al.* 2005) and follistatin (gene symbol: FST); and/or the BMP/GDF/SMAD1/5/8 signalling pathway is inhibited by one or more compounds selected from the group consisting of 6-[4-[2-(1-Piperidinyl)ethoxy]phenyl]-3-(4-pyridinyl)-pyrazolo[1,5-a]pyrimidine dihydrochloride (dorsomorphin; Yu *et al.* 2008), noggin (gene symbol: NOG) and gremlin (gene symbol: GREM1). These molecules are well known in the art and are shown, for example, in Figure 18.

These compounds and recombinant proteins are well known in the art and are commercially available to the skilled person.

Preferred amounts of SB431542 to be employed are between 0.1 and 100 µM, even more preferably between 1 and 50 µM, such as for example between 5 and 25 µM, such as between 10 and 20 µM and most preferably the amount is 15 µM.

Preferred amounts of A-83-01 to be employed are between 0.01 and 10 µM, even more preferably between 0.1 and 5 µM, and most preferably the amount is 0.5 µM.

In another more preferred embodiment of the method of the invention, the BMP/GDF/SMAD1/5/8 signalling pathway is additionally inhibited.

Preferred amounts of dorsomorphin to be employed are between 0.01 and 10 µM, even more preferably between 0.1 and 5 µM, and most preferably the amount is 0.5 µM

Preferred amounts of recombinant Noggin to be employed are between 10 and 10000 ng/ml, more preferably between 100 and 1000 ng/ml, and most preferably the amount is between 200 and 500 ng/ml.

In accordance with this embodiment, not only the FGF2 signalling pathway is inhibited, but also both the nodal/activin/TGFbeta/SMAD2/3 signalling pathway and the BMP/GDF/SMAD1/5/8 signalling pathway.

In accordance with the present invention it was shown that inhibition of these additional two pathways serves accessory functions to the inhibition of FGF2 signaling for the induction of neuroectoderm development. Inhibition of TGFβ/SMAD2, in cooperation with FGF/ERK inactivation, led to a rapid down-regulation of *NANOG* expression and downstream hESC-specific genes (Fig. 3g). In the presence of FGF2, however, this was not sufficient to induce neuroectoderm development.

Furthermore, when FGF2 is withdrawn from the media or when the pathway is inhibited by pharmacologic agents, autocrine BMP signaling becomes activated enough to interfere with neuroectodermal commitment. Addition of Noggin (NOG) or dorsomorphin (DM) was therefore employed to prevent BMP-mediated effects, such as the activation of *HAND1* and the repression of *SOX2* (Fig. 3g).

In another preferred embodiment of the method of the invention, the expression and/or activity of FGFR, MEK and/or MAPK are inhibited.

Inhibitors of FGFR, MEK and/or MAPK are well known in the art and have been described, for example, in Bain et al., Biochem. J 408, 297-315, 2007(PD0325901), Mattingly et al., J. Pharmacol. Exp. Ther. 316, 456-465, 2006 (PD184352), Dudley et al., Proc.Natl.Acad.Sci.USA 92, 7686, 1995 (PD98059), Favata et al, J.Biol.Chem. 273, 18623, 1998 (U0126), Sun et al., J.Med.Chem. 42, 5120, 1999 (SU5402), and Bansal et al., J.Neurosci.Res. 74, 486, 2003 (PD173074).

In another preferred embodiment of the method of the invention, the activity of MEK is inhibited by one or more of the compounds selected from the group consisting of 2-(2-Chloro-4-iodo-phenylamino)-N-cyclopropylmethoxy-3,4-difluoro-benzamide (PD184352), N-[(2R)-2,3-dihydroxypropoxy]-3,4-difluoro-2-[(2-fluoro-4-iodophenyl)amino]-benzamide (PD0325901), 2'-Amino-3'-methoxyflavone (PD98059) and 1,4-diamino-2,3-dicyano-1,4-bis (2-aminophenylthio)butadiene (U0126); and/or wherein the activity of FGFR is inhibited by 2-[(1,2-Dihydro-2-oxo-3H-indol-3-ylidene)methyl]-4-methyl-1H-pyrrole-3-propanoic acid (SU5402) and/or N-[2-[[4-(Diethylamino)butyl]amino]-6-(3,5-dimethoxyphenyl)pyrido[2,3-d] pyrimidin-7-yl]-N'-(1,1-dimethylethyl)urea (PD173074). These molecules are well known in the art and are shown, for example, in Figure 18.

These compounds are well known in the art and are commercially available to the skilled person.

PD0325901 is commercially available from various manufacturers. Preferred amounts of PD0325901 to be employed are between 0.001 and 50 µM, even more preferably between 0.01 and 25 µM, such as for example between 0.1 and 10 µM, such as between 0.25 and 5 µM and most preferably the amount is between 0.5 and 1 µM.

PD184352 is commercially available from various manufacturers. Preferred amounts of PD184352 to be employed are between 0.001 and 50 µM, even more preferably between 0.01 and 25 µM, such as for example between 0.1 and 10 µM, such as between 0.25 and 5 µM and most preferably the amount is between 0.5 and 1 µM.

PD98059 is commercially available from various manufacturers. Preferred amounts of PD98059 to be employed are between 0.1 and 1000 µM, even more preferably between 1 and 100 µM, and most preferably the amount is between 10 and 50 µM.

U0126 is commercially available from various manufacturers. Preferred amounts of U0126 to be employed are between 0.1 and 200 µM, even more preferably between 1 and 100 µM, and most preferably the amount is between 5 and 20 µM.

SU5402 is commercially available from various manufacturers. Preferred amounts of SU5402 to be employed are between 0.1 and 200 µM, even more preferably between 1 and 100 µM, and most preferably the amount is between 5 and 20 µM.

PD173074 is commercially available from various manufacturers. Preferred amounts of PD173074 to be employed are between 0.001 and 10 µM, even more preferably between 0.01 and 1 µM, and most preferably the amount is between 0.05 and 0.2 µM.

In a further preferred embodiment of the method of the invention, said pluripotent cells are cultured in the form of embryoid bodies.

In accordance with the present invention, the term "embryoid bodies", also abbreviated herein as "EB", refers to cell aggregates derived from pluripotent stem cells. Embryoid bodies are generally comprised of a large variety of differentiated cell types. Cell aggregation is for example imposed by culture in suspension, such as for example as a hanging drop culture or other methods that prevent cells from adhering to a surface, thus allowing the embryoid bodies to form their typical colony growth. Upon aggregation, differentiation is typically initiated and the cells begin to a limited extent to recapitulate embryonic development.

Embryoid body formation may be induced and/or obtained prior to step (a) of the method of the present invention or, alternatively, the pluripotent cells may be cultured during step (a) under conditions leading to the formation of embryoid bodies. Preferably, embryoid body formation is induced and/or obtained prior to step (a), such as for example 1 day prior to step (a).

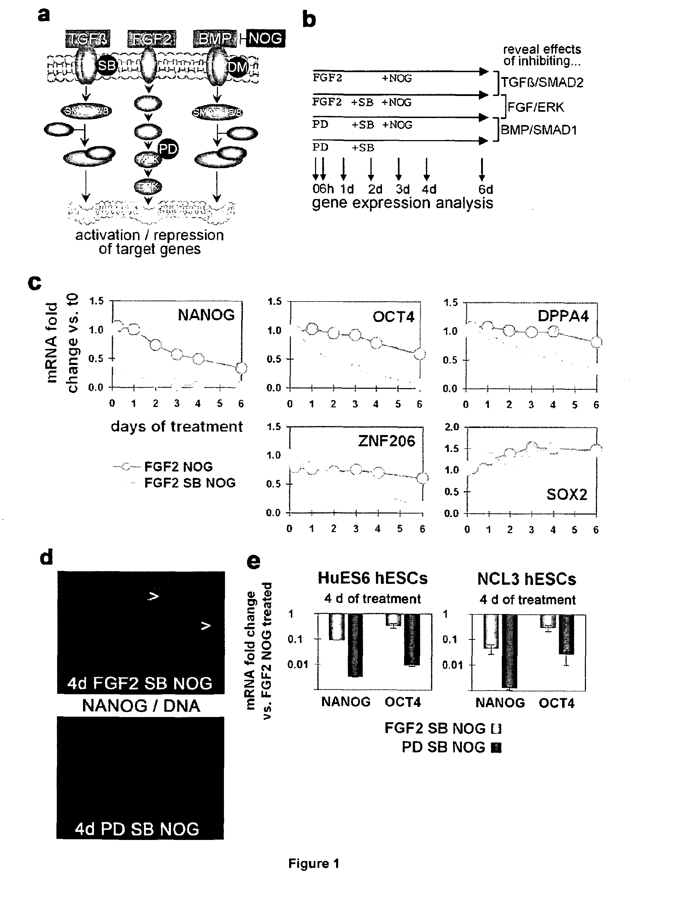

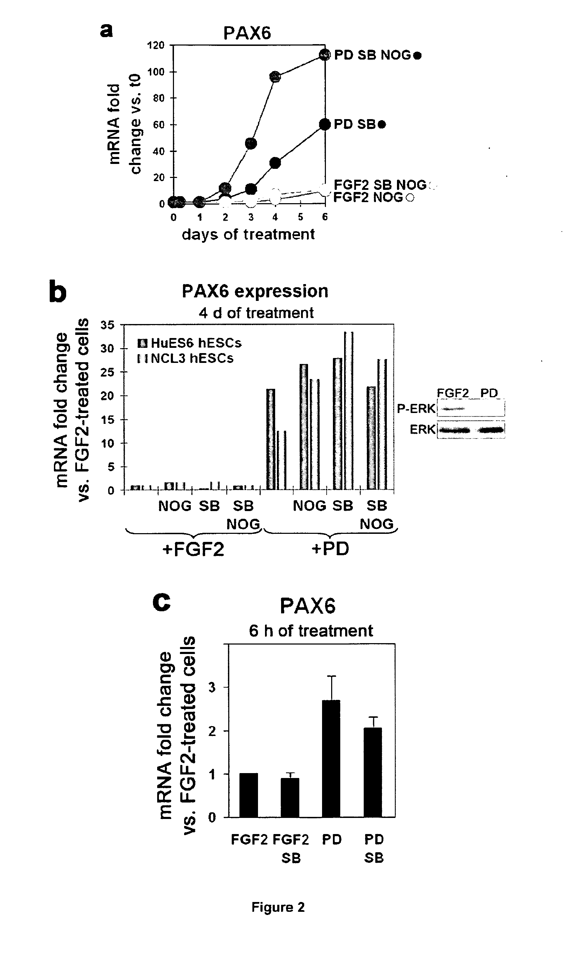

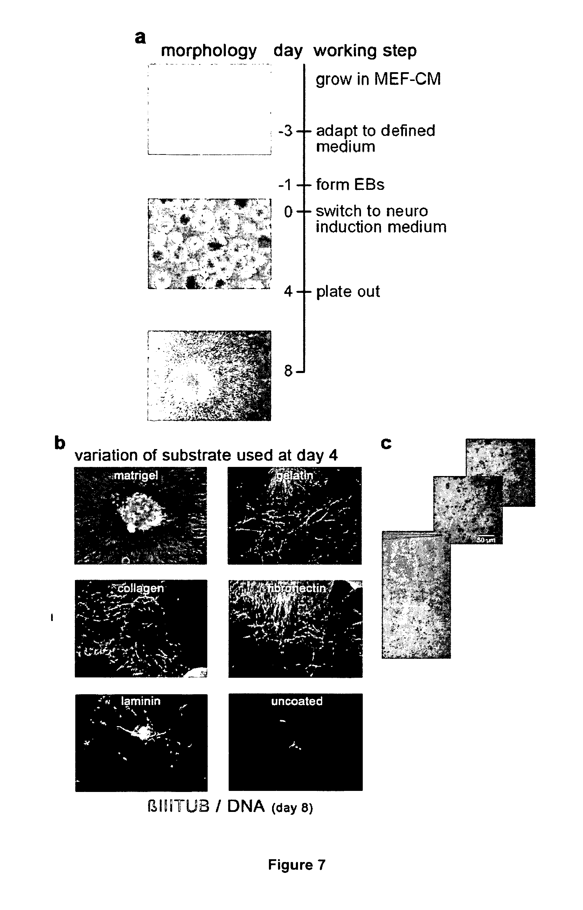

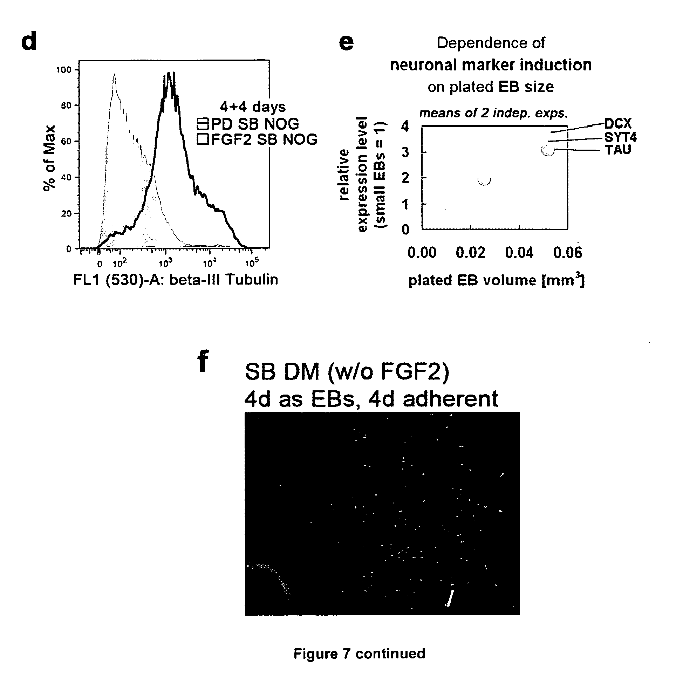

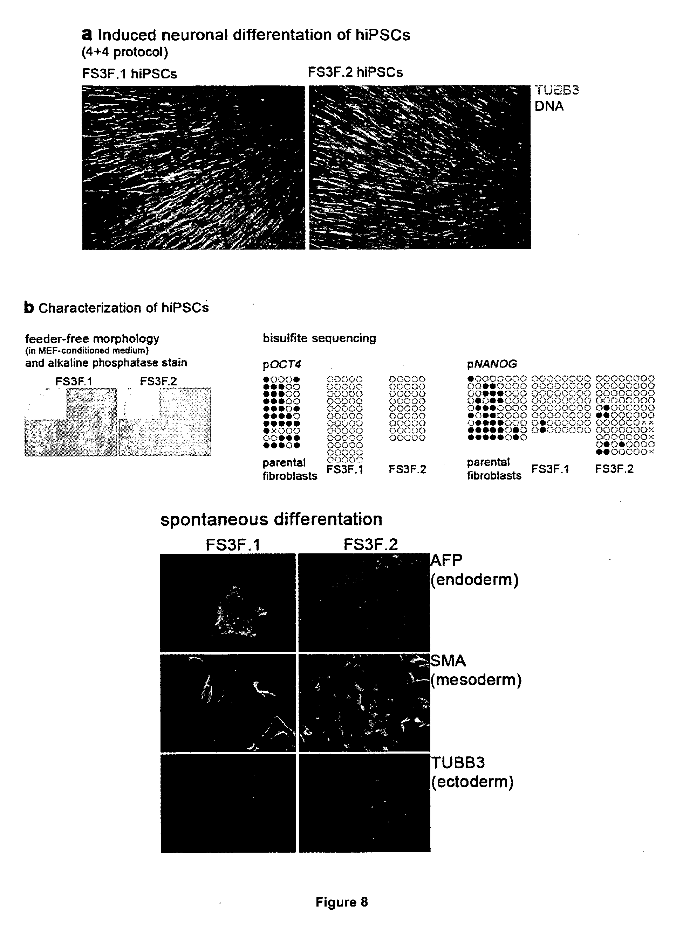

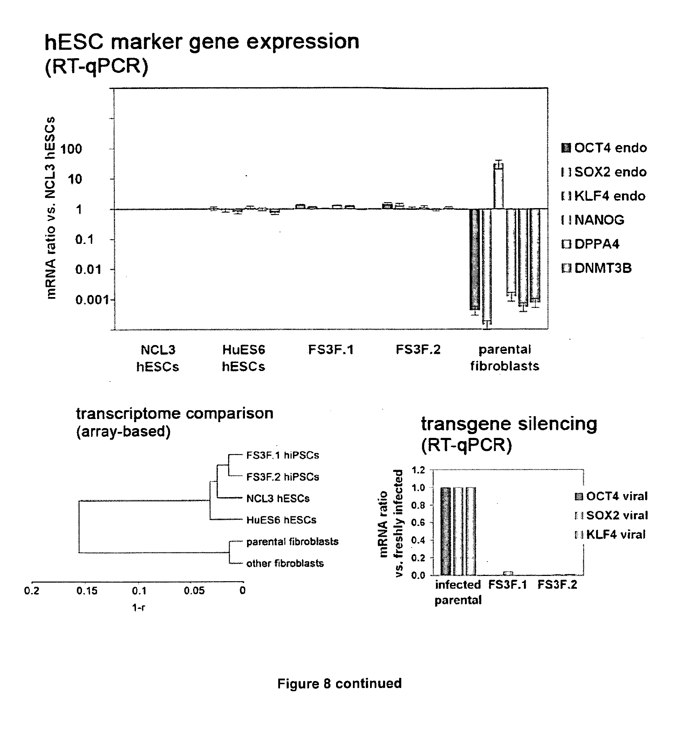

As is shown in the appended examples (Example 5), prolonged treatment of the neuroectoderm cells, such as e.g. 8 days in adherent culture, gave rise to neuron-like cells that stained positive for beta-III Tubulin. However, as these cultures still contained PAX6⁺ cells, a cell culture protocol was tested that involved neuroectoderm formation in embryoid bodies (EBs) for four days, followed by plating these on coated dishes for another four days ("4+4 protocol"; Fig. 7a). Strikingly, a large number of neuron-like cells was seen to grow out of the plated EBs, in a radial manner, and stained strongly positive for beta-III Tubulin (Fig. 2b). About two thirds (line HuES6) or up to 100% (line NCL3) of plated EBs displayed this pattern. Fluorescence-activated cell sorting analysis of bulk cultures indicated that most cells were positive for beta-III Tubulin, whereas there appeared to be a correlation between the size of the plated EBs and the efficiency of neuronal marker induction (Fig. 7d,e). This protocol was also compatible with substituting dorsomorphin (DM) for noggin (NOG) — i.e. with using a cocktail of low-cost small molecules only (Fig. 1a and Fig. 7f). Finally, when the protocol was applied to induced pluripotent stem cells (iPSCs), results were similar to those obtained with hESCs (Fig. 8).

Thus, by including a culture step wherein the cells are cultured in the form of embryoid bodies, the number of neuron-like cells differentiated from the neuroectoderm cells could be increased as compared to an exclusively adherent cell culture.

The present invention further relates to a method for generating neurons, the method comprising (a) the steps of the method of the invention defined herein above; and culturing the neuroectoderm cells of (a) for at least about 2 further days.

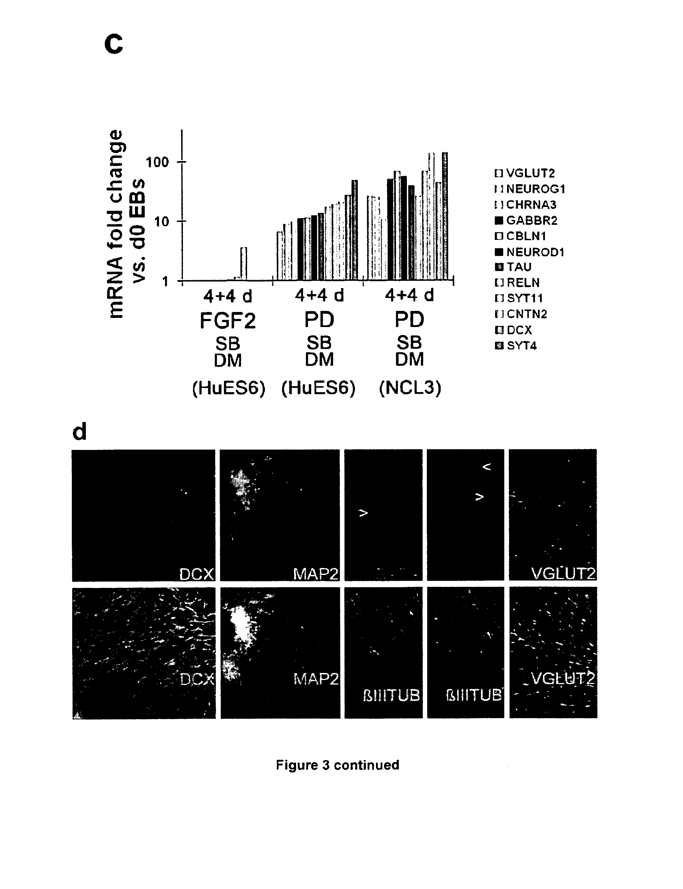

As described herein above and in the appended examples, prolonged treatment of the neuroectoderm cells in adherent culture gave rise to neuron-like cells that stained positive for beta-III Tubulin. Moreover, by inducing neuroectoderm formation in embryoid bodies in step (a), the number of neuron-like cells was further increased. Expression profiles of HuES6 and NCL3 4+4 cultures revealed a high enrichment of genes associated with neuronal differentiation but virtually no other type of lineage (Fig. 3c). In the periphery of the outgrowths, the cells stained positive for Doublecortin, a marker for immature neurons (Fig. 3d). MAP2 expression was confined to the intersection area between the centre and the periphery of the outgrowths. Only rarely did cells stain positive for GABA or tyrosine hydroxylase, suggesting some degree of specification into GABAergic and dopaminergic neurons, respectively. However, many cells stained positive for vesicular glutamate transporter 2, indicative of glutamatergic identity (Fig. 3d), a typical default fate of hESC-derived neurons (Li *et al.* 2009), which is also confirmed by pronounced VGLUT2 mRNA levels (Fig. 3c). Electrophysiological characterisation on days 8 and 12 using the whole cell patch clamp technique revealed the ability of the cells to fire one or more action potentials upon injecting depolarising currents, with an average amplitude of 69 ± 3 mV (n = 9, resting membrane potential: -44 ± 1.3 mV). Addition of tetrodotoxin (TTX) reversibly erased action potentials, demonstrating the functionality of fast voltage-gated sodium channels (Fig. 4e).

In a preferred embodiment of the method of generating neurons, the neurons are peripheral neurons.

The term "peripheral neurons", as used herein, relates to neurons of the peripheral nervous system, i.e. neurons that are part of the nervous system but are not in the brain or spinal cord. Peripheral neurons are usually associated with supplying sensation (i.e. sensory neurons) or motor (motor neurons) messages to the skin and skeletal muscles.

As is shown in Figures 14 and 15, the neurons generated with this method of the present invention are predominantly sensory neurons, i.e. a type of peripheral neurons. Furthermore, Figure 16 shows that the efficiency of generating sensory neurons (marked by BRN3A) using the method of the present invention is between 60 and 80% of all cells at day 8. To the inventors best knowledge, this is the most rapid and efficient protocol for sensory neuron generation to date.

In a more preferred embodiment of the method of generating neurons, the peripheral neurons are sensory neurons.

In a more preferred embodiment of the method of generating neurons, the method comprises after step (a) that the neuroectoderm cells are cultured in step (b) as an adherent cell culture.

"Adherent cell culture", as used herein, refers to a method of culture wherein the cells adhere to the solid support. Methods of growing cells as adherent culture are well known in the art. When grown as adherent cell culture, the cells may grow as monolayers, single cells or cell clusters.

The term "solid support", as used herein, refers to a surface enabling the adherence of cells thereto. Said surface may be, for example, the wall or bottom of a culture vessel, a plastic or glass slide such as for example a microscope slide or (a) bead(s) offering a surface for adherence. Conditions suitable to allow attachment of the cells are also well known to the skilled person and have been described, for example, in Schmitz, 2009. Preferably, said conditions are achieved by coating the solid support with an agent that enhances attachment of cells to the solid support. Such coating agents as well as methods of using them are also well known in the art and include, without being limiting, matrigel as for example described in the examples below, but also gelatine, fibronectin, poly-L-lysin, poly-L-ornithin, collagen, tenascin, perlecan, phosphocan, brevican, neurocan, thrombospondin and laminin. Most preferably, the solid support is coated with matrigel. Matrigel is well known in the art and is commercially available, for example from BD Biosciences.

In a further more preferred embodiment of the method of generating neurons, said neuroectoderm cells in step (b) are cultured for at least about 4 days.

In another more preferred embodiment of the method of generating neurons, the inhibition of the expression and/or activity of said one or more proteins selected from the group consisting of FGFR, SOS, GRB2, RAS, RAF, MEK and MAPK is maintained in step (b).

In accordance with this embodiment, the FGF2 signalling pathway that is inhibited in step (a) is further inhibited during the culture period in step (b). Preferably, the same protein(s) of the FGF2 signalling pathway that is inhibited in step (a) is further inhibited during the culture period in step (b). However, also envisaged herein is that (an) alternative protein(s) of the FGF2 signalling pathway selected from the group consisting of FGFR, SOS, GRB2, RAS, RAF, MEK and MAPK is inhibited in step (b). In other words, the protein(s) inhibited in step (b) may be the same or different from the protein(s) inhibited in step (a).

As is shown in the examples below, FGF2/ERK signaling also inhibits differentiation at a later stage (Fig. 3f). When the factors PD0325901 (PD), SB431542 (SB) and Noggin (NOG) were used to initiate neuroectoderm formation in embryoid bodies (e.g. by inhibition of all three pathways), and then various media were employed for plating these embryoid bodies after 4 days, it was found that continuous PD+SB+NOG treatment gave rise to a large number of beta-III Tubulin―positive neurons. In comparison, cells not treated with any factor yielded more heterogeneous cultures from day 5 onwards. In contrast, switching the medium from PD+SB+NOG to FGF+SB+NOG greatly reduced the number of beta-III Tubulin-positive cells obtained, confirming that FGF2 plays an inhibitory role in the second phase of differentiation (Fig. 3f).

In a preferred embodiment of the methods of the invention, the cells obtained are essentially free of pathogens, more preferably the cells are free of pathogens. Such pathogens are well known to the skilled person and include, without being limiting, viruses such as for example Hepatitis virus A, B, C, Epstein-Barr-Virus or HIV-Virus and bacteria such as for example mycoplasm or chlamydia.

The present invention further relates to a kit comprising means for inhibiting the expression and/or activity of one or more of the proteins selected from the group consisting of FGFR, SOS, GRB2, RAS, RAF, MEK and MAPK, and/or means inhibiting the nodal/activinTGFbeta/SMAD2/3 signalling pathway, and/or the BMP/GDF/SMAD1/5/8 signalling pathway and, optionally, pluripotent cells.

In its broadest sense, the term "kit" does not require the presence of any other compounds, vials, containers and the like. Preferably, the various components of the kit may be packaged in one or more containers such as one or more vials. Consequently, the various components of the kit may be present in isolation or combination. The containers or vials may, in addition to the components, comprise preservatives or buffers for storage.

"Means for inhibiting the expression and/or activity of one or more of the proteins selected from the group consisting of [...]" are well known in the art and include, without being limiting, any of the inhibitors as defined herein above.

The term "comprising" in the context of the kit of the invention denotes that further components can be present in the kit. Non-limiting examples of such further components include preservatives, buffers for storage, enzymes etc.

Also encompassed by this embodiment is that the kit comprises further means for inhibiting the expression and/or activity of one or more protein(s) different from the recited proteins. Such different proteins include, without being limiting, additional signalling proteins, such as for example Rho-associated, coiled-coil containing protein kinase (gene symbols ROCK1 and ROCK2), to increase overall cell survival (Watanabe et al., Nat. Biotech. 25, 681-686, 2007).

If the kit comprises such additional means for inhibiting the expression and/or activity of one or more protein(s) different from the recited proteins, it is preferred that at most 1.000 such additional means are comprised in the kit of the invention. More preferably, at most 500, such as for example at most 200 and more preferably at most 100 additional means are comprised in the kit of the invention. More preferably, at most 80, such as for example at most 60, more preferable at most 40, such as for example at most 30, at most 20, at most 10 and more preferably at most 8 additional means are comprised in the kit of the invention. Even more preferably, at most 5, such as for example at most 4, more preferable at most 3, such as for example at most 2 and yet more preferably at most 1 additional mean (s) is/are comprised in the kit of the invention. Also preferred is that the kit of the invention only comprises means for inhibiting the expression and/or activity of one or more of the proteins selected from the group as defined in accordance with the method of the present invention.

It is further preferred that the kit of the invention comprises instructions how to use the kit.

The definitions as well as the preferred embodiments provided herein above with regard to the method for generating neuroectoderm cells and the method for generating neurons apply *mutatis mutandis* also to this embodiments relating to a kit as outlined above.

Unless otherwise defined, all technical and scientific terms used herein have the same meaning as commonly understood by one of ordinary skill in the art to which this invention belongs. In case of conflict, the patent specification, including definitions, will prevail.

The figures show:
**Figure 1**: Inhibition of TGFβ/SMAD2 and FGF/ERK pathways cooperatively diminishes hESC-specific gene expression. (a) Simplified scheme of signaling pathways. Dark shading colour denotes pathway inhibition. Small molecule inhibitors are PD0325901 (PD), SB431542 (SB), and dorsomorphin (DM). (b) Design of microarray time-course experiment. Four time-series of hESCs treated in the indicated ways were monitored after 0 h, 6 h, 1 d, 2 d, 3 d, 4 d, and 6 d. (c) Array expression data for selected pluripotency genes in the hESC line HuES6. (d) Immunostaining for NANOG in the hESC line NCL3. Note that FGF2 SB NOG treatment did not inhibit all expression of NANOG in all cells but additional FGF/ERK inhibition did. (e) RT-qPCR analysis in the two hESC lines. Note the cooperation between SB and PD treatment in down-regulating *NANOG* as well as OCT4 expression.

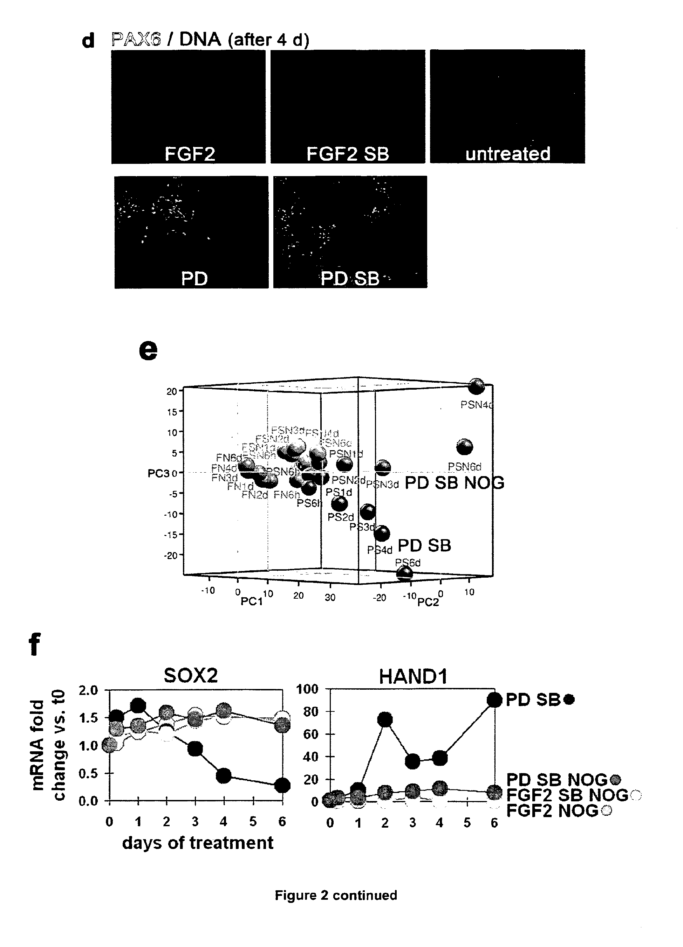

**Figure 2**: FGF/ERK inhibition induces *PAX6* and *HAND1* expression. (a) Time-course array data. Note that *PAX6* expression becomes induced only upon inhibition of the FGF/ERK cascade. (b) RT-qPCR analysis of *PAX6* expression in two hESC lines (HuES6 and NCL3; n = 1). Note that inactivation of FGF2/ERK signaling is necessary and sufficient to induce *PAX6* expression. Right: Western blot confirming inactivation of ERK in the presence of PD. (c) RT-qPCR analysis of HuES6 hESCs (n = 2) after 6 h of treatment. (d) FGF2 blocks *PAX6* induction (immunostaining). (e) 3D-PCA plot of time-course data reveal differences between PD SB― and PD SB NOG―treated samples. (f) Array expression data. PD SB treatment in the absence of Noggin leads to SOX2 repression and *HAND1* induction. (g) FGF/ERK inhibition activates autocrine BMP signaling (RT-qPCR, HuES6 hESCs, n = 2). (h) PAX6/HAND1 immunostaining. With PD SB NOG treatment, PAX6⁻ cells were observed to be confined to the boundaries of the colonies.

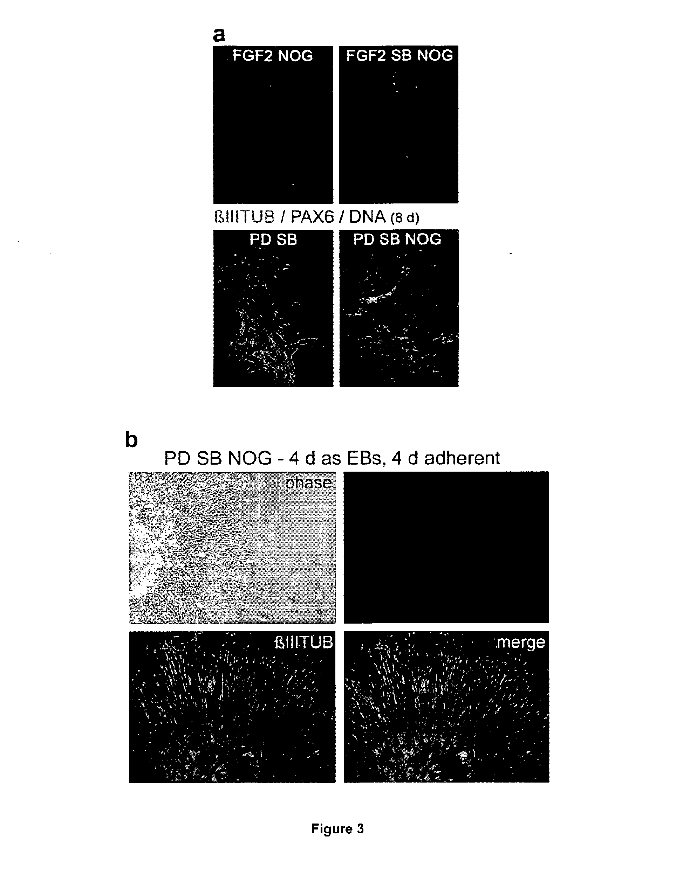

**Figure 3**: FGF2 inhibits neuronal differentiation of hESCs. (a) Immunostaining for beta-III Tubulin (TUBB) and PAX6 (HuES6). The number of TUBB⁺ cells obtained varied in independent experiments. (b) Beta-III Tubulin staining 4 d after plating PD SB NOG―treated EBs (8 d of treatment in total, HuES6). (c) In the 4+4 protocol, FGF2 prevents induction of neuronal genes (array data). (d) Immunocytochemical characterization of d 8 neurons obtained with the 4+4 protocol (NCL3 hESCs). (e) Electrophysiological characterization of NCL3-derived neurons on d 12 using whole cell patch clamping. Top left: Voltage responses to current injections causing fast, large amplitude action potentials. Top right and bottom: Generation of successive action potentials was reversibly inhibited using TTX. Similar data were obtained with HuES6 and at earlier time points (d 8). (f) FGF2 also inhibits neuronal differentiation when added after neuroectoderm formation (beta-III Tubulin staining, NCL3). (g) Model of gene regulation by three pathways in hESCs. Dashed lines indicate unknown molecular mechanisms.
**Figure 4**: Time-course array data of the expression of selected pluripotency genes. PD SB NOG treatment generally led to a more rapid down-regulation of gene expression compared with FGF2 SB NOG treatment. Note that at the 6 h time point, NANOG expression was already down-regulated, consistent with a "direct" activation by the TGFβ/SMAD2 and FGF/ERK pathways. OCT4 down-regulation appeared to be delayed, suggesting that indirect regulatory mechanisms were acting on this gene.

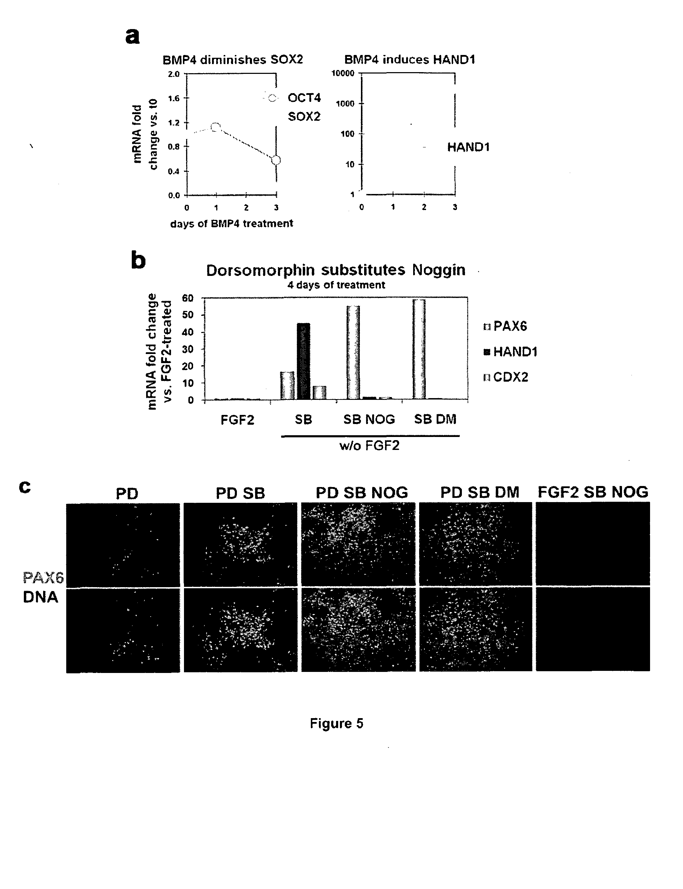

**Figure 5**: BMP signaling antagonizes neuroectodermal induction. (a) BMP stimulation analyzed by RT-qPCR (HuES6, n = 2). Note that SOX2 (light grey) becomes down-regulated before OCT4 (dark grey). (b) Dorsomorphin can replace Noggin in the neuroectodermal induction of hESCs. RT-qPCR analysis with HuES6 hESCs (n = 1). SB treatment in the absence of FGF2 leads to both *PAX6* (left column) and *HAND1* (middle column) induction. Addition of either Noggin or dorsomorphin abolished *HAND1* up-regulation in favour of *PAX6* induction. In the right column, CDX2 expression is shown. (c) PAX6 immunostaining after 4 d with the indicated treatments (HuES6). Note the enhanced percentage of PAX6⁺ cells when autocrine BMP signaling was suppressed.

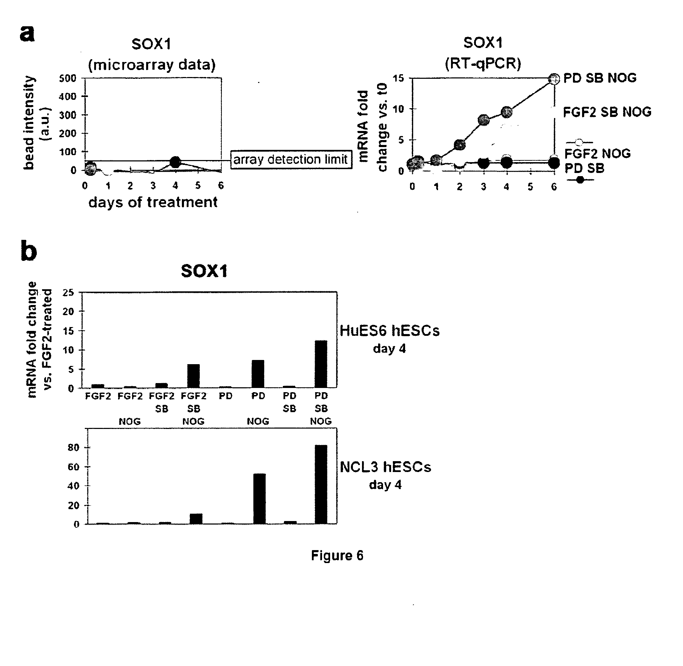

**Figure 6**: Transcriptional activation of *SOX1.* (a) *SOX1* mRNA levels were undetectable by microarray analysis of the time-course samples (left). Right: RT-qPCR analysis (HuES6). (b) RT-qPCR analysis with all possible treatment conditions in two hESC cell lines on d 4. Based on these data, it is concluded that inactivation of autocrine BMP signaling is necessary but not sufficient for the initial induction of *SOX1,* although, the corresponding Ct values were high in all conditions tested and SOX1 was not detected by immunocytochemistry, indicating that expression levels were in the sub-physiological range.
**Figure 7**: 2-step neuronal differentiation protocol. (a) Schematic showing the different steps in the protocol and corresponding phase contrast morphology. (b) Use of matrigel as a substrate for plating PD SB NOG―treated EBs yielded superior numbers and percentages of neurons staining positive beta-III Tubulin on d 8 (HuES6). (c) Dye injection of a d-12 neuron on matrigel shows long processes, some several hundred µm in length (NCL3). (d) FACS analysis of bulk cultures harvested d 8 (NCL3) for beta-III Tubulin. FGF2 SB NOG-treated cells served as biological negative control. The antibody concentration was not optimised. (e) RT-qPCR analysis for neuronal markers on d 8 (NCL3). Larger EBs (diameter > 300µm) plated on d 4 (see scheme in part a) tended to give rise to neurons at somewhat higher efficiency. (f) Dorsomorphin could substitute for Noggin in the neuroinduction medium, yielding neurons at comparable rates (NCL3).
**Figure 8**: Induced neuronal differentiation of hiPSCs. (a) Two foreskin fibroblast-derived hiPSC lines (reprogrammed using 3 factors: OCT4, SOX2, and KLF4) gave rise to beta-III Tubulin―positive cells at frequencies comparable to those obtained with hESCs. (b) Characterization of hiPSC lines used in part a, according to standard assays. Bottom left: Unlike the induced protocol outlined in Figure 7a and employed in part a, spontaneous differentiation of hiPSCs yielded beta-III Tubulin―positive cells at considerably lower efficiency. Compounds are listed in the legend in the same order as shown in the graphs.
**Figure 9**: At day 4, the neuroectodermal induction protocol using TGFβ/BMP inhibition with FGF/ERK inhibition in accordance with the present invention results in an approximately twice as efficient up-regulating in PAX6 mRNA expression as compared to TGFβ/BMP inhibition alone (as described in Chambers et al., Nat Biotech, 2009). The left columns shows results obtained for HuES6 cells, the right columns show results for NCL3 cells.
**Figure 10**: Neuroectodermal markers other than PAX6 (OTX2, OTX1, NR2F2) are efficiently induced by the method of the present invention using TGFβ/BMP inhibition with FGF/ERK inhibition using PD+SB+NOG.

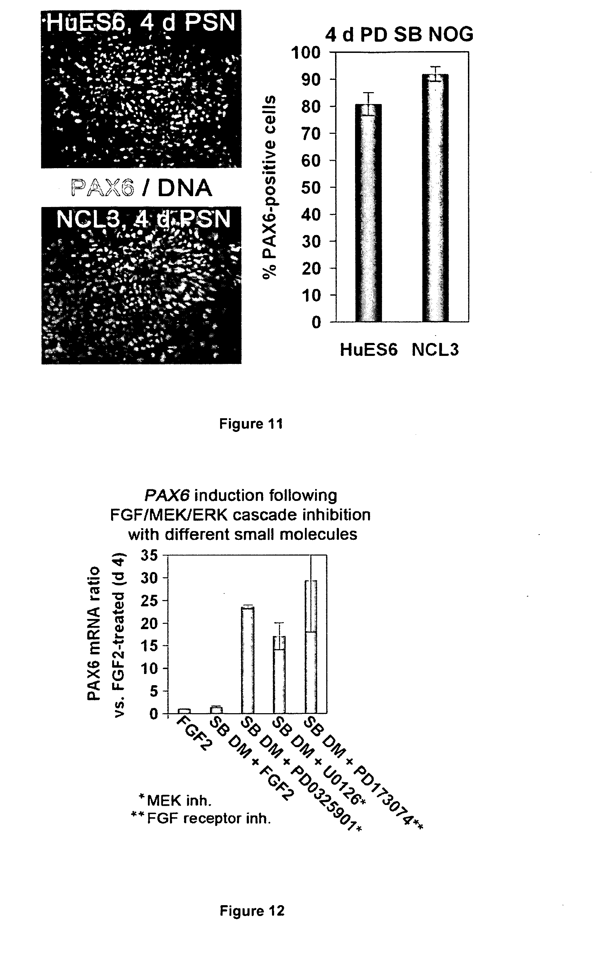

**Figure 11**: HuES6 and NCL3 cells cultured for four days in PD+SB+DM in accordance with the present invention resulted in a yield of between 80 and >90% PAX6-positive cells at day 4.
**Figure 12**: *PAX6* induction following FGF/MEK/ERK cascade inhibition with different small molecules. Replacement of PD0325901 with alternative inhibitors such as U0126 or PD173074 results in efficient up-regulation of *PAX6* mRNA after four days of treatment.

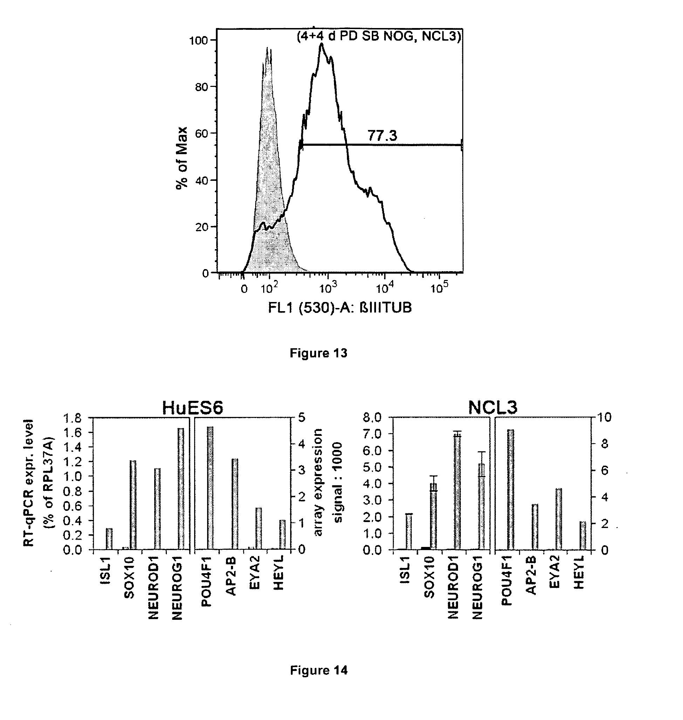

**Figure 13**: (βIIITUB-positive cells at day 8. The overall yield of neurons generated by the 4+4 protocol (PD+SB+NOG; NCL3 cells, Figure 7a) is approx. 80%.
**Figure 14**: Neurons generated with the 4+4 protocol (PD+SB+DM; HuES6 and NCL3 cells, Figure 7a) are predominantly sensory neurons (a type of peripheral neurons), as shown by the expression of neural crest and sensory neuron markers. Embryoid bodies at day 0 are shown in the left columns, neurons generated after 4+4 days according to the method of the invention in PD+SB+DM are shown the right columns, respectively.

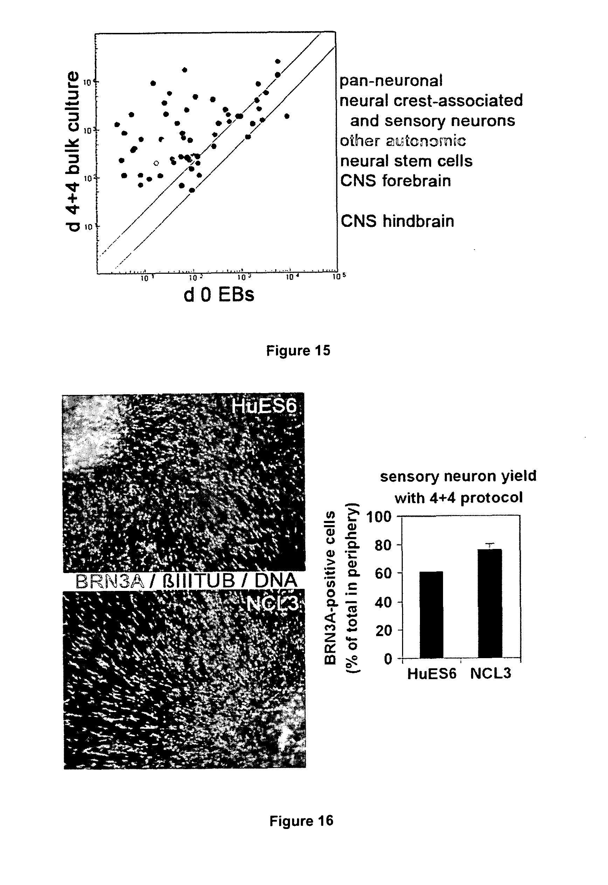

**Figure 15**: Neurons generated with the 4+4 protocol (PD+SB+DM; HuES6 and NCL3 cells, Figure 7a) are predominantly sensory neurons (a type of peripheral neurons) as shown by neuronal subtype analysis in the cell line NCL3.
**Figure 16**: Sensory neuron yield. The efficiency of generating sensory neurons (marked by BRN3A) using the 4+4 protocol outlined in Figure 7a is between 60 and 80% of all cells at day 8.
**Figure 17**: Growth factor requirements between days 4 to 8 of the 4+4 protocol (after EB plating). FGF2 addition suppresses neuron formation, Smad1/Smad2 inhibition is dispensable, and continuous FGF/ERK suppression is required between days 4 to 8 for optimal neuronal differentiation (superior to untreated conditions, see chart). (A) 4 days growth as EB in PD+SB+NOG, then 4 days adherent in the conditions shown. (B) TUBB3 expression after 4 days growth as EB in PD+SB+DM, then 4 days adherent in the conditions shown.
**Figure 18**: Structures of suitable inhibitors.

The examples illustrate the invention:

### Example 1: Methods and Materials

### Cell culture.

Fully characterised hESC lines HuES6 (Cowan *et al.* 2004) and NCL3 (Zhang *et al*. 2006) were received through the Harvard Office of Technology Development and the UK Stem Cell Bank, respectively, and used at a passage number less than 50. Both lines were confirmed to give rise to derivatives of all three germ layers by spontaneous *in vitro* differentiation. hESCs tested negative for mycoplasma. All experiments under adherent conditions were carried out using matrigel-coated 6- or 12-well dishes. Matrigel coating was important for both maintenance of self-renewal and efficient neuronal differentiation: Matrigel HC (growth factor reduced, BD) was thawed on ice overnight and then diluted 1:3 with ice-cold Knockout DMEM (Invitrogen). 1 ml stocks were then kept frozen at -20 °C. For coating of plates, each 1 ml stock was re-suspended in 24 ml of conventional hESC medium (Amit *et al.* 2004) (without growth factors) on ice (1:75 total dilution, final protein concentration: - 0.25 mg/ml). Pre-cooled 6- or 12-wells were covered with 1 ml or 0.4 ml of diluted matrigel, respectively. Plates were then wrapped in parafilm and kept at room temperature overnight. Next day, plates were transferred to 4 °C for at least one day. Before use, coating quality was verified under a phase contrast cell culture microscope.

hESCs were routinely maintained in MEF-conditioned medium (Xu *et al.* 2001) and split using colony pre-cutting with sterile injection needles, followed by treatment with dispase (2 mg/ml), repeated washing in PBS with Mg²⁺/Ca²⁺, lifting off the cell clumps using a sharp plastic scraper (PAA), and several seconds of centrifugation to collect the aggregates without single cells. Experiments were exclusively conducted in chemically defined medium. hESCs were always pre-adapted to defined medium for at least 2 d (see Fig. 7a). Defined medium—modified from Yao *et a*/*.* 2006 —contained DMEM/F12 (Hyclone), 1x N2 supplement, 1x B27 supplement, 0.1 mM ß-mercaptoethanol (Invitrogen), 1x nonessential amino acids, 1x L-glutamine, 1x penicillin/streptomycin (PAA), and 0.2% human serum albumin (Biological Industries). For maintenance of self-renewal, N2NB27 medium was supplemented with 20 ng/ml of FGF2 (Peprotech) or FGF2 plus 0.25 µM of dorsomorphin. Upon splitting, 10 µM of ROCK inhibitor (Watanabe *et al.* 2007) (Ascent) was additionally supplemented. Growth factor or inhibitor treatments were typically initiated 1 d after splitting: FGF2 (20 ng/ml), Noggin (Peprotech, 250 ng/ml), BMP4 (Peprotech, 20 ng/ml), SB431542 (Sigma, 15 µM), PD0325901 (Axon Medchem, 0.5―1 µM), dorsomorphin (Merck, 0.5 µM). For embryoid body formation, cell aggregates were collected as for routine splitting and then transferred to bacteriological 6 cm dishes containing defined medium with FGF2 and ROCK inhibitor. For media changes, EBs were collected in the centre of the dishes by slowly rotating the dishes under a stereo microscope. EBs were then transferred to new dishes with minimal liquid. Upon neuroectodermal induction of EBs, addition of ROCK inhibitor for the first 3 d was found to significantly improve cell survival without interfering with the overall experimental outcome. Medium was changed daily in all experiments.

### RT-qPCR and microarray analysis.

Cells were lysed directly in culture dishes and RNA was isolated using RNeasy kits with on-column DNA digestion (QIAGEN). RT-qPCR and microarray analyses were carried out as described (Greber *et al.* 2010), using fully validated primers with normalisation to two housekeeping genes and HumanRef-8 v3 bead chips (Illumina), respectively. Principal component analysis was performed using an in-house developed algorithm in Matlab.

### Immunocytochemistry, Western blotting, and FACS.

For immunocytochemistry, cells in 12-well dishes were fixed with 4% paraformaldehyde for 10 min, permeabilised with 0.2% Triton X-100 for 10min, and blocked with a solution comprising 2% BSA, 5% FCS, and 0.1 % Tween 20 in PBS for 45 min. Primary and secondary antibodies were applied overnight at 4°C and for 1 h at room temperature, respectively, in 0.5% BSA in PBS-T. Hoechst stain was applied in the second-to-last washing step at 1 µg/ml. Samples were mounted with Aqua-Poly/Mount (Polysciences) and round glass cover slips. Western blotting was performed according to standard procedures. FACS analysis was performed using an intracellular staining kit (Fix & Perm, Caltag) and BD FACSAria instrumentation. Primary antibodies were: α AFP (Sigma A8452, 1:500); α Doublecortin (Santa Cruz sc-8067, 1:100); α GABA (Sigma A2052, 1:1,000); α HAND1 (Santa Cruz sc-9413, 1:25); α MAP2 (Millipore MAB3418, 1:200); α MAPK Cell Signaling Technology 9102, 1:1,000); α NANOG (R&D AF1997, 1:50); α PAX6 (Covance PRB-278P, 1:500); α Phospho-MAPK (Cell Signaling Technology 9106, 1:2,000); α SMA (DakoCytomation M0851, 1:100); α beta-III Tubulin (Sigma T8660, 1:1,000; Covance PRB-435P 1:2,000; BD Pharmingen 560381, 20 µl per sample); α TH (Millipore AB152, 1:250); α VGLUT2 (Millipore MAB5504, 1:50). Secondary Alexa 488, 568, and 594 labeled antibodies were diluted 1:1,000 (catalog numbers A21468, A21206, A11078, A11008, A11001, A11079, A11061, A11011—all from Invitrogen). Unless otherwise stated, representative pictures are shown.

### Electrophysiology.

Recordings were performed in culture dishes at room temperature and at 33 °C. Cell cultures were super-fused with a solution containing 120 mM NaCl, 2.5 mM KCI, 1.25 mM NaH₂PO₄, 30 mM HEPES, 2 mM MgSO₄, 2 mM CaCl₂, 10 mM dextrose, 25 µM Alexa-594 hydrazide; pH: 7.25 with HCl; osmolality: 305 mOsmol/kg. Electrodes for whole cell recordings with a resistance of 6-8 MΩ were pulled from borosilicate glass capillaries. Electrodes were filled with a saline solution containing 95 mM K-gluconate, 20 mM K₃-citrate, 10 mM NaCl, 10 mM HEPES, 1 mM MgCl₂, 0.5 mM CaCl₂, 3 mM BAPTA, 3 mM Mg-ATP, 0.5 mM Na-GTP; pH: 7.25 with KOH; osmolality: 295 mOsm/kg. Electrodes were connected to an EPC-10 amplifier (HEKA Elektronik, Lamprecht, Germany). After gaining access to the cell, dyes were allowed to equilibrate by diffusion for at least 5 min. Access resistances were in the range of 5-20 MΩ. Experiments were controlled by PatchMaster software (HEKA Elektronik). Liquid junction potential was not corrected for. Tetrodotoxin (0.5―1 µM) was applied via bath perfusion to block Na⁺ channels and abolish action potentials.

### hiPSC generation.

Human iPSCs were produced from foreskin fibroblasts (ATCC #CRL-2097) by following Melton's protocol (Huangfu *et al*. 2008) using 3 factors (OCT4, SOX2, KLF4). Retroviruses were produced in 293T cells using Fugene 6 (Roche) and Addgene plasmids 8454, 8449, 17217, 17218, and 17219 (Takahashi *et al.* 2007). Two lines that showed typical hESC morphology and growth characteristics were further characterized according to standard assays (Fig. 8b). Bisulfite sequencing was carried out using an EpiTect Bisulfite kit (Qiagen), followed by PCR cloning and sequencing of inserts.

### Example 2: Inhibition of FGF2, TGFβ/SMAD2 and BMP/SMAD1 pathways

Recombinant proteins and pharmacological inhibitors were used to selectively activate or repress the activity of the FGF2 signaling pathway, the TGFβ/SMAD2 and the BMP/SMAD1 pathways (Fig. 1a). Four types of treatment conditions were designed to monitor the effects of the individual pathways on global gene expression over a period of 6d (Fig. 1b). As a baseline, hESCs were treated in chemically defined medium with FGF2 and the BMP antagonist Noggin (= FGF2 NOG), a condition that promotes self-renewal (Xu *et a*/*.* 2005). A comparison with FGF2 SB NOG treated cells is suitable to reveal the transcriptional response of SMAD2 inactivation (Fig. 1a). A comparison of FGF2 SB NOG versus PD SB NOG treatment, instead, is suitable to reveal effects of the FGF/ERK cascade. Finally, a comparison of PD SB NOG with PD SB treatment is suitable to clarify the role of BMP inactivation (Fig. 1 b).

A prerequisite for hESC differentiation is the shutdown of the self-renewal machinery. Analysis of the time-course data confirmed that FGF2 NOG treatment largely sustained the expression of pluripotency markers. A decline in the expression of certain markers was observed toward the end of the time-course, which could be due to the near confluency of the cultures on day 6 (d 6). In contrast, additional supplementation with SB led to a strong decline in the expression of self-renewal markers over the course of 6 d, indicating a loss of hESC identity (Fig. 1c). NANOG was down-regulated very rapidly, consistent with the finding that it is a direct target of SMAD2 (Greber, Lehrach and Adjaye 2008; Xu *et al*. 2008; Vallier *et al.* 2009a). It is likely, therefore, that the other members of the hESC self-renewal network, such as OCT4, were affected through the down-regulation of NANOG. Interestingly, expression of SOX2, which is not only required by undifferentiated hESCs but also by neuroectoderm, was maintained with FGF2 SB NOG treatment (Fig. 1c). This configuration - low *NANOG* and *OCT4* but high *SOX2* levels ― is compatible with neuroectoderm formation. However, a time point-by-time point comparison of the two conditions revealed that only a few genes were actually becoming induced by SB treatment. Based on this observation, it is concluded that inactivation of TGFβ/SMAD2 signaling is rather effective in down-regulating the expression of hESC-specific genes but that in the presence of FGF2 and NOG, inhibition of TGFß/SMAD2 signaling is not sufficient to activate the expression of prominent differentiation-associated markers.

Despite these negative effects of SB treatment on hESC self-renewal, immunocytochemistry analysis revealed that some cells still stained positive for NANOG after 4 d in culture (Fig. 1d, top). FGF/ERK signaling in hESCs has been suggested to maintain NANOG expression in cooperation with TGFß/SMAD2 (Greber *et al.* 2010). We therefore compared the down-regulation kinetics of hESC markers with FGF2 SB NOG versus PD SB NOG treatment. More effective down-regulation of prominent hESC markers was found with PD SB NOG compared with FGF2 SB NOG. An 80% down-regulation in NANOG mRNA levels was seen within 6 h of treatment with PD SB NOG; NANOG mRNA levels were undetectable by d 4 (Fig. 4 and Fig. 1d, bottom). The co-operativity between inactivation of SMAD2 and ERK signaling was confirmed by RT-qPCR in two hESC lines—HuES6 and NCL3—on d 4 (Fig. 1e). It was therefore concluded that inhibition of both TGFß/SMAD2 and FGF/ERK signaling enables most effective down-regulation of the expression of hESC-specific genes and the effective maintenance of SOX2 expression (Fig. 4).

### Example 3: Induction of neuroectodermal markers

Next, the induction of neuroectodermal markers was investigated with an emphasis on *PAX6* (Zhang *et al.* 2010). A time-course comparison revealed a pronounced up-regulation of *PAX6* in the PD SB― and PD SB NOG―treated samples, but not in the FGF2 NOG― or FGF2 SB NOG―treated samples, revealing the presence of a strong association between FGF/ERK inhibition and *PAX6* induction (Fig. 2a). This result was confirmed by performing RT-qPCR with samples that had been treated for 4 d with all possible combinations of molecules (Fig. 2b). Interestingly, a moderate up-regulation in *PAX6* mRNA levels was already detectable within 6 h of FGF/ERK inhibition, suggesting the presence of an immediate-early regulatory link (Fig. 2c). Immunocytochemistry analysis confirmed the suppression of *PAX6* induction by FGF2, whereas many ― albeit not all ―cells grown in the absence of FGF2 or with FGF/ERK inhibition showed pronounced PAX6 staining (Fig. 2d). These data show that FGF2/ERK signaling represses *PAX6* expression in hESCs. This effect must be independent of its *NANOG-*maintaining function, as SB treatment was as effective in reducing NANOG expression but SB plus FGF2 treatment did not induce *PAX6* expression.

### Example 4: The accessory effect of BMP/SMAD1 pathway inhibition

Global time-course analysis suggested a high overall similarity in gene expression changes between the PD SB― and PD SB NOG―treated samples. 3D principal component analysis, however, suggested differences in a subset of genes (Fig. 2e). Strikingly, SOX2 expression was not well maintained with PD SB, whereas *HAND1,* encoding a basic helix-loop-helix transcription factor involved in extra-embryonic as well as mesodermal development (Firulli *et al.* 1998; Riley *et al.* 1998), was strongly induced in the absence of NOG (Fig. 2f). These effects are reminiscent of the activation of BMP signaling in hESCs (Greber *et al.* 2008). The rapid suppression of SOX2 concomitant with induction of *HAND1* by BMP4 stimulation was confirmed using an independent hESC line (Fig. 5a). It was thus hypothesized that FGF/ERK suppression has ambivalent effects on neuroectodermal induction: On the one hand, it leads to *NANOG* down-regulation as well as *PAX6* induction (productive). On the other hand, it activates autocrine BMP/SMAD1 signaling (Greber, Lehrach and Adjaye 2007; Peerani *et al.* 2007), which may induce non-neural differentiation (Li *et al.* 2007) (counterproductive). Indeed, *HAND1,* which is a BMP/SMAD1 target, became induced within only 6 h of FGF/ERK inactivation, whereas additional NOG supplementation could rescue this effect (Fig. 2g). Hence, FGF/ERK inhibition may cause differentiation into different types of lineages ― neuroectodermal or extra-embryonic/mesodermal. Indeed, hESCs treated with PD or PD SB stained heterogeneously positive for both PAX6 and HAND1. However, addition of NOG suppressed the induction of *HAND1* in favor of *PAX6* expression (Fig. 2h). Based on these data, it was concluded that the addition of NOG enhances the differentiation into neuroectodermal cell types by antagonising the BMP/SMAD1 activating effect of FGF/ERK inhibition. Hence, in this context, NOG acts as a specificity enhancer for neuroectodermal commitment, consistent with its role in model organisms (Gaulden and Reiter 2008).

This interpretation was confirmed by substituting NOG with a small molecule inhibitor of BMP/SMAD1 signaling, dorsomorphin (Yu *et al.* 2008), yielding pronounced PAX6 expression in most cells by d 4 (Fig. 5b,c). The neuroectodermal identity of this population was confirmed by the pronounced induction of other markers, such as *OTX1* and *OTX2,* peaking on d 4. SOX1, another prominent transcription factor, was undetectable by microarrays or immunocytochemistry. Only some low-level transcriptional induction was seen using RT-qPCR (Fig. 6). Consistent with this result is the absence of SOX1 in early human neuroectoderm, as recently reported (Zhang *et al.* 2010).

### Example 5: Development of neuronal cells

It was further investigated whether prolonged treatment would give rise to neuron-like cells, as in mouse epiblast stem cells (Greber *et al.* 2010). Indeed, after 8 d in adherent culture, cells staining positive for beta-III Tubulin were readily observed. However, these cultures still contained many PAX6⁺ cells, indicating asynchronous differentiation (Fig. 3a). A modified protocol was thus employed that involved neuroectoderm formation in embryoid bodies (EBs) for 4 d, followed by plating these on matrigel-coated dishes for another 4 d ("4+4 protocol", Fig. 7a). Strikingly, a large number of neuron-like cells was seen to grow out of the plated EBs, in a radial manner, and stained strongly positive for beta-lll Tubulin (Fig. 2b). About two thirds (line HuES6) or up to 100% (line NCL3) of plated EBs displayed this pattern. Matrigel appeared to be a particularly preferred substrate for the outgrowth of these cells for, on d 12, some of the cellular processes (presumably neurites) grew up to several hundred µm in length (Fig. 7b,c). Fluorescence-activated cell sorting analysis of bulk cultures indicated that most cells were positive for beta-lll Tubulin, whereas there appeared to be a correlation between the size of the plated EBs and the efficiency of neuronal marker induction (Fig. 7d,e). The protocol was also compatible with substituting DM for NOG ― i.e. with using a cocktail of low-cost small molecules only (Fig. 1 a and Fig. 7f). Finally, when the protocol was applied to induced pluripotent stem cells (iPSCs), results were similar to those obtained with hESCs (Fig. 8).

Assessment of the expression profiles of the HuES6 and NCL3 4+4 cultures revealed a high enrichment of genes associated with neuronal differentiation but virtually no other type of lineage (Fig. 3c). In the periphery of the outgrowths, the cells stained positive for Doublecortin, a marker for immature neurons (Fig. 3d). MAP2 expression was rather confined to the intersection area between the centre and the periphery of the outgrowths. Only rarely did cells stain positive for GABA or tyrosine hydroxylase, suggesting some degree of specification into GABAergic and dopaminergic neurons, respectively. However, many cells stained positive for vesicular glutamate transporter 2 indicative of glutamatergic identity (Fig. 3d), a typical default fate of hESC-derived neurons (Li *et al.* 2009), which is also confirmed by pronounced VGLUT2 mRNA levels (Fig. 3c). Electrophysiological characterisation on d 8 and 12 using whole cell patch clamp revealed the ability of the cells to fire one or more action potentials upon injecting depolarizing currents, with an average amplitude of 69 ± 3 mV (n = 9, resting membrane potential: -44 ± 1.3 mV). Addition of tetrodotoxin (TTX) reversibly erased action potentials, demonstrating the functionality of fast voltage-gated sodium channels (Fig. 4e).

### References

Amit, M., Shariki, C., Margulets, V. & Itskovitz-Eldor, J. Feeder layer- and serum-free culture of human embryonic stem cells. Biol Reprod 70, 837-845 (2004).
Bottcher, R. T. & Niehrs, C. Fibroblast growth factor signaling during early vertebrate development. Endocr Rev 26, 63-77 (2005).
Chambers, S. M. et al. Highly efficient neural conversion of human ES and iPS cells by dual inhibition of SMAD signaling. Nat Biotechnol 27, 275-280 (2009).
Conti, L. et al. Niche-independent symmetrical self-renewal of a mammalian tissue stem cell. PLoS Biol 3, e283 (2005).
Cowan, C. A. et al. Derivation of embryonic stem-cell lines from human blastocysts. N Engl J Med 350, 1353-1356 (2004).
Feng and Derynck. Specificity and versatility in tgf-beta signaling through Smads. Annu Rev Cell Dev Biol, 2005, Vol 21, 659-693-
Firulli, A. B., McFadden, D. G., Lin, Q., Srivastava, D. & Olson, E. N. Heart and extra-embryonic mesodermal defects in mouse embryos lacking the bHLH transcription factor Hand1. Nat Genet 18, 266-270 (1998).
Gaulden, J. & Reiter, J. F. Neur-ons and neur-offs: regulators of neural induction in vertebrate embryos and embryonic stem cells. Hum Mol Genet 17, R60-66 (2008).
Gerrard, L., Rodgers, L. & Cui, W. Differentiation of human embryonic stem cells to neural lineages in adherent culture by blocking bone morphogenetic protein signaling. Stem Cells 23, 1234-1241 (2005).
Greber, B., Lehrach, H. & Adjaye, J. Fibroblast growth factor 2 modulates transforming growth factor beta signaling in mouse embryonic fibroblasts and human ES cells to support hESC self-renewal. Stem Cells 25, 455-464 (2007).
Greber, B., Lehrach, H. & Adjaye, J. Control of early fate decisions in human ES cells by distinct states of TGFbeta pathway activity. Stem Cells Dev 17, 1065-1077 (2008).
Greber, B. et al. Conserved and divergent roles of FGF signaling in mouse epiblast stem cells and human embryonic stem cells. Cell Stem Cell 6, 215-226 (2010).
Huangfu, D. et al. Induction of pluripotent stem cells from primary human fibroblasts with only Oct4 and Sox2. Nat Biotechnol 26, 1269-1275 (2008).
Kang, H. B. et al. Basic fibroblast growth factor activates ERK and induces c-fos in human embryonic stem cell line MizhES1. Stem Cells Dev 14, 395-401 (2005).
Koch, P., Opitz, T., Steinbeck, J. A., Ladewig, J. & Brustle, O. A rosette-type, self-renewing human ES cell-derived neural stem cell with potential for in vitro instruction and synaptic integration. Proc Natl Acad Sci U S A 106, 3225-3230 (2009).
Kunath, T. et al. FGF stimulation of the Erk1/2 signalling cascade triggers transition of pluripotent embryonic stem cells from self-renewal to lineage commitment. Development 134, 2895-2902 (2007).
Laping et al. Inhibition of transforming growth factor (TGF)-beta1-induced extracellular matrix with a novel inhibitor of the TGF-beta type I receptor kinase activity: SB-431542. Mol. Pharmacol. 62, 58-64, (2002)
LaVaute, T. M. et al. Regulation of neural specification from human embryonic stem cells by BMP and FGF. Stem Cells 27, 1741-1749 (2009).
Li, J. et al. MEK/ERK signaling contributes to the maintenance of human embryonic stem cell self-renewal. Differentiation 75, 299-307 (2007).
Li, X. J. et al. Coordination of sonic hedgehog and Wnt signaling determines ventral and dorsal telencephalic neuron types from human embryonic stem cells. Development 136, 4055-4063 (2009).
Murry, C. E. & Keller, G. Differentiation of embryonic stem cells to clinically relevant populations: lessons from embryonic development. Cell 132, 661-680 (2008).
Pera, M. F. et al. Regulation of human embryonic stem cell differentiation by BMP-2 and its antagonist noggin. J Cell Sci 117, 1269-1280 (2004).
Peerani, R. et al. Niche-mediated control of human embryonic stem cell self-renewal and differentiation. Embo J 26, 4744-4755 (2007).
Riley, P., Anson-Cartwright, L. & Cross, J. C. The Hand1 bHLH transcription factor is essential for placentation and cardiac morphogenesis. Nat Genet 18, 271-275 (1998).
Rosa, A., and Brivanlou, A.H. A regulatory circuitry comprised of miR-302 and the transcription factors OCT4 and NR2F2 regulates human embryonic stem cell differentiation. Embo Journal 30, 237-248 (2011).
Schmierer, B. & Hill, C. S. TGFbeta-SMAD signal transduction: molecular specificity and functional flexibility. Nat Rev Mol Cell Biol 8, 970-982 (2007).
Simeone, A. et al. A vertebrate gene related to orthodenticle contains a homeodomain of the bicoid class and demarcates anterior neuroectoderm in the gastrulating mouse embryo. Embo Journal 12, 2735-2747 (1993).
Smith, J. R. et al. Inhibition of Activin/Nodal signaling promotes specification of human embryonic stem cells into neuroectoderm. Dev Biol 313, 107-117 (2008).
Stavridis, M. P., Lunn, J. S., Collins, B. J. & Storey, K. G. A discrete period of FGF-induced Erk1/2 signalling is required for vertebrate neural specification. Development 134, 2889-2894 (2007).
Takahashi, K. et al. Induction of pluripotent stem cells from adult human fibroblasts by defined factors. Cell 131, 861-872 (2007).
Tesar, P. J. et al. New cell lines from mouse epiblast share defining features with human embryonic stem cells. Nature 448, 196-199 (2007).
Tojo et al., The ALK-5 inhibitor A-83-01 inhibits Smad signaling and epithelial-to-mesenchymal transition by transforming growth factor-β. Cancer.Sci. 96, 791 (2005).
Vallier, L. et al. Early cell fate decisions of human embryonic stem cells and mouse epiblast stem cells are controlled by the same signalling pathways. PLoS One 4, e6082 (2009).
Vallier, L. et al. Activin/Nodal signalling maintains pluripotency by controlling Nanog expression. Development 136, 1339-1349 (2009a).
Watanabe, K. et al. A ROCK inhibitor permits survival of dissociated human embryonic stem cells. Nat Biotechnol 25, 681-686 (2007).
Xu, C. et al. Feeder-free growth of undifferentiated human embryonic stem cells. Nat Biotechnol 19, 971-974 (2001).
Xu, R. H. et al. Basic FGF and suppression of BMP signaling sustain undifferentiated proliferation of human ES cells. Nat Methods 2, 185-190 (2005).
Xu, R. H. et al. NANOG is a direct target of TGFbeta/activin-mediated SMAD signaling in human ESCs. Cell Stem Cell 3, 196-206 (2008).
Yao, S. et al. Long-term self-renewal and directed differentiation of human embryonic stem cells in chemically defined conditions. Proc Natl Acad Sci U S A 103, 6907-6912 (2006).
Ying, Q. L., Stavridis, M., Griffiths, D., Li, M. & Smith, A. Conversion of embryonic stem cells into neuroectodermal precursors in adherent monoculture. Nat Biotechnol 21, 183-186 (2003).
Yu, P. B. et al. Dorsomorphin inhibits BMP signals required for embryogenesis and iron metabolism. Nat Chem Biol 4, 33-41 (2008).
Zhang, S. C., Wernig, M., Duncan, I. D., Brustle, O. & Thomson, J. A. In vitro differentiation of transplantable neural precursors from human embryonic stem cells. Nat Biotechnol 19, 1129-1133 (2001).
Zhang, X. et al. Derivation of human embryonic stem cells from developing and arrested embryos. Stem Cells 24, 2669-2676 (2006).
Zhang, X. et al. Pax6 Is a Human Neuroectoderm Cell Fate Determinant. Cell Stem Cell 7, 90-100 (2010).

## Claims

1. A method for generating neuroectoderm cells, the method comprising:
culturing pluripotent cells for about 2 to about 6 days, wherein the expression and/or activity of one or more proteins selected from the group consisting of fibroblast growth factor receptor (FGFR), son of sevenless (SOS), growth factor receptor-bound protein 2 (GRB2), rat sarcoma protein (RAS), rat fibrosarcoma (RAF), mitogen-activated protein kinase kinase (MEK) and mitogen-activated protein kinase (MAPK) is inhibited.

2. The method of claim 1, wherein said human pluripotent cells are cultured for at least about 4 days.

3. The method of claim 1 or 2, wherein additionally
the nodal/activin/tumour growth factor beta/small mothers against decapentaplegic 2/3 (Nodal/activin/TGFbeta/SMAD2/3) signalling pathway; and/or
the bone morphogenic protein/growth differentiation factor/small mothers against decapentaplegic 1/5/8 (BMP/GDF/SMAD1/5/8) signalling pathway is inhibited.

4. The method of claim 3, wherein
the nodal/activin/TGFbeta/SMAD2/3 signalling pathway is inhibited by one or more compounds selected from the group consisting of 4-[4-(3,4-Methylenedioxyphenyl)-5-(2-pyridyl)-1H-imidazol-2-yl]-benzamide hydrate (SB431542), 3-(6-Methylpyridin-2-yl)-1-phenylthiocarbamoyl-4-quinolin-4-ylpyrazole (A-83-01) and follistatin; and/or
the BMP/GDF/SMAD1/5/8 signalling pathway is inhibited by one or more compounds selected from the group consisting of 6-[4-[2-(1-Piperidinyl)ethoxy]phenyl]-3-(4-pyridinyl)-pyrazolo[1,5-a]pyrimidine dihydrochloride (dorsomorphin), noggin and gremlin.

5. The method of claim 3 or 4, wherein the BMP/GDF/SMAD1/5/8 signalling pathway is additionally inhibited.

6. The method of any one of claims 1 to 5, wherein the expression and/or activity of FGFR, MEK and/or MAPK are inhibited.

7. The method of any one of claims 1 to 6, wherein the activity of MEK is inhibited by one or more of the compounds selected from the group consisting of 2-(2-Chloro-4-iodo-phenylamino)-N-cyclopropylmethoxy-3,4-difluoro-benzamide (PD184352), N-[(2R)-2,3-dihydroxypropoxy]-3,4-difluoro-2-[(2-fluoro-4-iodophenyl)amino]-benzamide (PD0325901), 2'-Amino-3'-methoxyflavone (PD98059) and 1,4-diamino-2,3-dicyano-1,4-bis (2-aminophenylthio)butadiene (U0126); and/or wherein the activity of FGFR is inhibited by 2-[(1,2-Dihydro-2-oxo-3H-indol-3-ylidene)methyl]-4-methyl-1 H-pyrrole-3-propanoic acid (SU5402) and/or N-[2-[[4-(Diethylamino)butyl]amino]-6-(3,5-dimethoxyphenyl)pyrido[2,3-d]pyrimidin-7-yl]-N'-(1,1-dimethylethyl)urea (PD173074).

8. The method of any one of claims 1 to 7, wherein said pluripotent cells are cultured in the form of embryoid bodies.

9. A method for generating neurons, the method comprising
the steps of the method according to any one of claims 1 to 8; and
culturing the neuroectoderm cells of (a) for at least about 2 further days.

10. The method of claim 9, wherein after step (a) the neuroectoderm cells are cultured in step (b) as an adherent cell culture.

11. The method of claim 9 or 10, wherein said neuroectoderm cells in step (b) are cultured for at least about 4 days.

12. The method of any one of claims 9 to 11, wherein the inhibition of the expression and/or activity of said one or more proteins selected from the group consisting of FGFR, SOS, GRB2, RAS, RAF, MEK and MAPK is maintained in step (b).

13. A kit comprising means for inhibiting the expression and/or activity of one or more of the proteins selected from the group consisting of FGFR, SOS, GRB2, RAS, RAF, MEK and MAPK, and/or means inhibiting the nodal/activinTGFbeta/SMAD2/3 signalling pathway, and/or the BMP/GDF/SMAD1/5/8 signalling pathway and, optionally, pluripotent cells.
